# EUROPEAN PATENT SPECIFICATION

(11) **EP 1 365 240 B1**
(45) Date of publication and mention of the grant of the patent: **13.09.2006**
(21) Application number: 03011084.5
(22) Date of filing: 21.05.2003
(51) Int. Cl.: G01N 33/53, G01N 33/543

(54) **Immunoassay methods, immunoassay apparatuses, and reagents for immunoassays**
Immunologische Verfahren, Vorrichtungen und Reagenzien
Procédés, appareils et réactifs immunologiques

(30) Priority: 22.05.2002 JP 2002147085
(43) Date of publication of application: 26.11.2003
(73) Proprietor: SYSMEX CORPORATION, Hyogo 651-0073 (JP)
(72) Inventor: Kawate, Yasunori, Kakogawa-shi, Hyogo 675-0104 (JP)
(74) Representative: HOFFMANN EITLE

(56) References cited:
- EP-A- 0 126 450
- EP-A- 0 174 195
- EP-A- 1 387 171
- WO-A-00/49385
- WO-A-01/96868
- GB-A- 2 123 146
- US-A- 5 405 784

## Description

### BACKGROUND

The present invention relates to immunoassay methods, immunoassay apparatuses, and reagents for immunoassay using particle agglutination of carrier particles immobilized with an antigen or antibody against a substance contained in a specimen such as blood, urine, ascites, or the like.

Immunoassay methods to detect a certain substance in a specimen such as blood by taking advantage of an antigen-antibody reaction have been used in the field of clinical laboratory examinations. Examples include radio immunoassay (RIA), enzyme immunoassay (EIA), fluoroimmunoassay (FIA), and particle agglutination. In the particle agglutination method, a specimen believed to contain a measuring object substance is blended with carrier particles immobilized with an antibody or antigen responding to the measuring object substance. Since the carrier particles are agglutinated by the antigen-antibody reaction if the measuring object substance exists in the specimen, their agglutination is detected optically.

Serum or plasma obtained by eliminating blood cells from whole blood is generally used as the specimen for immunological examination; however, if particles such as erythrocytes, fat particles and bacteria have not been eliminated, they influence optical detection of the carrier particle agglutination. In addition, whole blood contains large amounts of erythrocytes; thus, if whole blood is used as the specimen, they also influence optical detection of the carrier particle agglutination. However, in an emergency examination, an assay using whole blood that does not require the additional step of blood cell elimination is desirable.

An immunoassay method enabling the use of whole blood based on a counting immunoassay using carrier particles different in size than the erythrocytes is described in International Publication WO 01/96868. However, in this method, it is necessary to select carrier particles that do not overlap in size with erythrocytes.

US 5,405,784 relates to an immune agglutination method based on different homogenous populations of fluorescent beads specifically interacting with one or more ligands. The specimen and bead mixture is analyzed based on a single parameter, fluorescence per ligand, to determine the number of nonagglutinated beads and the number of agglutinated beads.

In the patent application GB 2 123 146 A a dual parameter flow immunoassay with particles having a detectable different property such as size and fluorescence is disclosed. The formation of aggregates of the two different kinds of particles is measured by detection of particles with mixed properties.

Moreover, various parameters such as infection-related substances, tumor-related substances and hormones have been used as measuring parameters in immunological examinations. A multiple parameter automatic analysis apparatus has become popular for use in immunological examinations, and typically involves the following steps: a specimen is dispensed into multiple reaction vessels depending on the number of measuring parameters; reagents are dispensed according to the specific measuring parameters; the prescribed reaction treatments are performed successively; and respective reaction samples are measured. Such multiple parameter automatic analysis apparatuses require many measuring reagents and many reaction vessels, which results in large and complicated designs.

In addition, the amount of specimen required for an examination increases with the number of measuring parameters, thereby increasing the burden on a patient. Thus, an immunoassay method for simultaneously measuring multiple parameters in one sample has been desirable. An example of such an immunoassay method is described in Japanese unexamined patent publication sho61-132870. In this method, a reagent and carriers such as latex having different particle diameters are immobilized with multiple types of antibodies. An antibody labeled with a labeling substance and specimens are reacted such that they become an immune complex of antibody immobilized carrier-antigens of the measuring object substance-labeled antibodies. Particles with labeling information are analyzed based on particle diameter by measuring parameters and obtaining information for particle diameter and labeling substance.

However, in the above-described method, each carrier does not necessarily form an immune complex, and agglutination of the carriers occurs by reacting the antigen of the measuring object substance with antibodies of multiple carriers. Moreover, latex particles cause agglutination by the influence of surface charges. Once the carriers are agglutinated, the apparent particle diameter becomes large, and differentiation of the measuring parameters based on particle diameters becomes impossible. Furthermore, as a reagent for detection of the antigen, an antibody labeled with a labeling substance is required in addition to the carrier particles immobilized with the antibody responding to the antigen.

### SUMMARY

The scope of the present invention is defined solely by the appended claims, and is not affected to any degree by the statements within this summary.

In one embodiment, the invention relates to an immunoassay method comprising:
(a) preparing a measuring sample by mixing a specimen and carrier particles;
   wherein the specimen comprises a measuring object substance and coexisting particles;
   wherein an antibody or an antigen against the measuring object substance is immobilised on the carrier particles;
   wherein the carrier particles have a different physical property than the coexisting particles;
   wherein said different physical property is selected from fluorescence, absorbance, phosphorescence, chemiluminescence or biological luminescence, and
   wherein the carrier particles are agglutinated when the measuring object substance is present in the specimen;
(b) detecting first and second optical information from the coexisting particles and the carrier particles in the measuring sample, wherein said first optical information is selected from fluorescence, absorbance, phosphorescence, chemiluminescence or biological luminescence, and said second optical information is scattered light;
(c) differentiating the carrier particles from the coexisting particles on the basis of the first optical information;
(d) detecting an agglutination level of the carrier particles on the basis of the second optical information; and
(e) analyzing whether the measuring object substance is present in the specimen or not on the basis of the agglutination level of the carrier particles.

In a further embodiment the invention relates to an immunoassay method comprising:
(a) preparing a measuring sample by mixing a specimen comprising first and second measuring object substances, and coexisting particles, and first and second carrier particles;
   wherein an antibody or an antigen against the first measuring object substance is immobilized on the first carrier particles, and the first carrier particles agglutinate when the first measuring object substance is present;
   wherein an antibody or an antigen against the second measuring object substance is immobilized on the second carrier particles, and the second carrier particles agglutinate when the second measuring object substance is present;
   wherein the first and second carrier particles comprise different optical information than the coexisting particles; and
   wherein the first carrier particles have a different physical property than the second carrier particles, wherein said different physical property is selected from fluorescence, absorbance, phosphorescence, chemiluminescence or biological luminescence;
(b) detecting the first and second optical information from the first and second carrier particles and the coexisting particles in the measuring sample,
   wherein said first optical information is selected from fluorescence, absorbance, phosphorescence, chemiluminescence or biological luminescence, and said second optical information is scattered light;
(c) differentiating the first carrier particles, the second carrier particles, and the coexisting particles on the basis of the first optical information;
(d) detecting respective agglutination levels of the first and second carrier particles on the basis of the second optical information; and
(e) analyzing whether at least one of the first and second measuring object substances is present in the specimen or not on the basis of the respective agglutination levels.

Moreover the invention relates to an immunoassay apparatus comprising:
(a) a measuring sample provider for providing a measuring sample wherein the measuring sample is a mixture of a specimen and carrier particles;
   wherein the specimen comprises a measuring object substance and coexisting particles;
   wherein an antibody or an antigen against the measuring object substance is immobilized on the carrier particles;
   wherein the carrier particles have a different physical property than the coexisting particles,
   wherein said different physical property is selected from fluorescence, absorbance, phosphorescence, chemiluminescence or biological luminescence; and
   wherein the carrier particles are agglutinated when the measuring object substance is present in the specimen;
(b) an optical information detector for detecting first and second optical information from the coexisting particles and the carrier particles in the measuring sample, wherein said first optical information is selected from fluorescence, absorbance, phosphorescence, chemiluminescence or biological luminescence, and said second optical information is scattered light;
(c) a controller which differentiates the carrier particles from the coexisting particles on the basis of the first optical information, calculates an agglutination level of the carrier particles on the basis of the second optical information, and analyzes whether the measuring object substance is present in the specimen or not on the basis of the agglutination level of the carrier particles.

In a further embodiment the invention relates to an immunoassay apparatus comprising:
(a) a measuring sample provider for providing a measuring sample wherein the measuring sample is a mixture of a specimen and first and second carrier particles,
   wherein the specimen comprises first and second measuring object substances and coexisting particles;
   wherein an antibody or an antigen against the first measuring object substance is immobilized on the first carrier particles, and the first carrier particles agglutinate when the first measuring object substance is present;
   wherein an antibody or an antigen against the second measuring object substance is immobilized on the second carrier particles, and the second carrier particles agglutinate when the second measuring object substance is present; and
   wherein the first carrier particles have a different physical property, than the second carrier particles, wherein said different physical property is selected from fluorescence, absorbance, phosphorescence, chemiluminescence or biological luminescence;
(b) an optical information detector for detecting the first and second optical information from the first and second carrier particles, wherein said first optical information is selected from fluorescence, absorbance, phosphorescence, chemiluminescence or biological luminescence, and said second optical information is scattered light;
(c) a controller which differentiates the first carrier particles, the second carrier particles and the coexisting particles on the basis of the first optical information, calculates respective agglutination levels of the first and second carrier particles on the basis of the second optical information; and analyzes whether at least one of the first and second measuring object substances is present in the specimen or not on the basis of the respective agglutination levels.

### BRIEF DESCRIPTION OF THE DRAWINGS

Fig. 1 shows a two dimensional scattergram where carrier particles and particles other than the carrier particles contained in a specimen emerged at different locations.
Fig. 2 shows a histogram of the particles emerged in the regions set on the two dimensional scattergram.
Fig. 3 shows a two dimensional scattergram in which the first and second carrier particles emerged at different locations.
Fig. 4 shows a histogram of the particles emerged in the regions set on the two dimensional scattergram.
Fig. 5 shows a perspective view of an automatic immunoassay apparatus embodying features of the present invention.
Fig. 6 shows an illustration of an internal configuration of an automatic immunoassay apparatus embodying features of the present invention.
Fig. 7 shows a perspective view of a sample preparing portion of an automatic immunoassay apparatus embodying features of the present invention.
Fig. 8 shows an exploded perspective view of a light detecting portion of an automatic immunoassay apparatus embodying features of the present invention.
Fig. 9 shows a schematic illustration of a control portion of an automatic immunoassay apparatus embodying features of the present invention.
Fig. 10 shows a schematic illustration of a total control of an automatic immunoassay apparatus embodying features of the present invention.
Fig. 11 shows a schematic illustration of a control of an analysis portion of an automatic immunoassay apparatus embodying features of the present invention.
Fig. 12 shows an illustration of a screen displayed on a liquid crystal touch panel of an automatic immunoassay apparatus embodying features of the present invention.
Fig. 13 shows a schematic illustration of a configuration of a flow cytometer used in accordance with the present invention.
Fig. 14 shows a two dimensional scattergram obtained in accordance with the present invention.
Fig. 15 shows a histogram obtained in accordance with the present invention.
Fig. 16 shows a two dimensional scattergram obtained in accordance with the present invention.
Fig. 17 shows a two dimensional scattergram obtained in accordance with the present invention.
Fig. 18 shows a obtained in accordance with the present invention.
Fig. 19 shows a histogram obtained in accordance with the present invention.
Fig. 20 shows a histogram obtained in accordance with the present invention.
Fig. 21 shows a histogram obtained in accordance with the present invention.
Fig. 22 shows a two dimensional scattergram obtained in accordance with the present invention.
Fig. 23 shows a two dimensional scattergram obtained in accordance with the present invention.
Fig. 24 shows a histogram obtained in accordance with the present invention.
Fig. 25 shows a histogram obtained in accordance with the present invention.
Fig. 26 shows a histogram obtained in accordance with the present invention.
Fig. 27 shows a histogram obtained in accordance with the present invention.
Fig. 28 shows a two dimensional scattergram obtained in accordance with the present invention.
Fig. 29 shows a two dimensional scattergram obtained in accordance with the present invention.
Fig. 30 shows a histogram obtained in accordance with the present invention.
Fig. 31 shows a histogram obtained in accordance with the present invention.
Fig. 32 shows a histogram obtained in accordance with the present invention.
Fig. 33 shows a histogram obtained in accordance with the present invention.
Fig. 34 shows a perspective view of a modified example of the sample preparing portion shown in Fig. 7.

### DETAILED DESCRIPTION OF THE PRESENTLY PREFERRED EMBODIMENTS

The present invention provides immunoassay methods, apparatuses, and reagents in which particles other than a measuring object substance that may be contained in a specimen have minimal effect while allowing high flexibility in selecting the size of carrier particles used in a particle agglutination immunoassay method.

In addition, the present invention provides immunoassay methods, apparatuses, and reagents capable of simultaneously measuring multiple parameters from one sample and of measuring without requiring a labeled antibody.

The immunoassay method of the present invention uses particle agglutination methods. Carrier particles that may be used in accordance with the present invention include those that are generally used in the particle agglutination methods including but not limited to latex particles, polystyrene particles, magnetic particles, glass particles, dendrimers, and the like, and combinations thereof.

With regard to an antigen or an antibody immobilized on the carrier particles, if a measuring object substance is an antibody, an antigen which specifically reacts to the antibody is used. If a measuring object substance is an antigen, an antibody which specifically reacts to the antigen is used. For example, if the measuring parameter is CEA antigen (cancer embryonic antigen), anti-CEA antibody is immobilized. If the measuring parameter is anti-HBs antibody, HBs antigen is immobilized.

In accordance with the present invention, a physical property is detected from the carrier particles. The carrier particles are differentiated from particles other than the measuring object substance contained in the specimen on the basis of a detected physical property. When multiple types of carrier particles are used in order to detect multiple measuring object substances, each type of particle is differentiated by the physical properties detected from the carrier particles. The physical property is optical information selected from forward scattered light, side scattered light, fluorescence, absorbance, phosphorescence, chemiluminescence and biological luminescence. A flow cytometer is an example of an apparatus that may be used to detect such optical information. This apparatus detects optical information such as fluorescent intensity, forward scattered light intensity and side scattered light intensity detected from respective particles by flowing a sample containing the carrier particles into a flow cell, irradiating laser light thereupon, and receiving fluorescence and forward scattered light generated when the carrier particle cuts across the laser light followed by photo-electric transfer. The apparatus can utilize voltage changes which occur when the particle passes through a micropore by making the particle pass through the micropore at which voltage is applied as a physical property. In accordance with the present invention, multiple physical properties as described above are detected from each particle, and differentiation of the particles and detection of the agglutination levels are carried out by the combination thereof as set out in the claims.

When using multiple optical information as the source of multiple physical properties, the carrier particles are differentiated from other particles in the specimen on the basis of the first optical information, wherein said first optical information is selected from fluorescence, absorbance, phosphorescence, chemiluminescence or biological luminescence. A fluorescent dye, dyestuff, luminescent substrate, or the like is appropriately contained on the carrier particles. Thus, high signal intensity occurs from the carrier particles by use of fluorescence, absorbance, phosphorescence, chemiluminescence, or biological luminescence. Since particles other than the measuring object substances-for example, erythrocytes, platelets and chylomicrons-are low in intensity of fluorescence, absorbance, phosphorescence, chemiluminescence, or biological luminescence, they are differentiated from the carrier particles by the first optical information. Thus, in the present invention, since the differentiation of particles is performed without the use of physical properties reflecting sizes of the particles, there is an advantage that fewer restrictions are required when selecting the sizes of the carrier particles. For instance, when the immunoassay is carried out using a specimen containing platelets, even if the carrier particles have sizes similar to those of the platelets being used, the carrier particles can be differentiated from the platelets by physical properties such as fluorescence detected from the carrier particles. In this case, since a physical property of the carrier particle per se is detected, it is not necessary to use an additional labeled antibody or the like.

The second optical information is used for the detection of agglutination levels of the carrier particles. The second optical information is scattered light. The phrase "agglutination level" refers to the degree of particle agglutination based on an antigen-antibody reaction. When information reflecting the apparent sizes of carrier particles, i.e. scattered light, in particular forward scattered light, is used as the second optical information, non-agglutinated carrier particles can be distinguished from agglutinated carrier particles and the agglutination level of the carrier particles can be obtained. If evenly spherical particles, such as latex particles, are used as the carrier particles, side scattered light can similarly be used as the information reflecting the sizes of the carrier particles. As this agglutination level, it is possible to use an agglutination rate obtained by the following method: (a) obtain the scattered light intensity of respective particles by flow cytometry; (b) differentiate the non-agglutinated single particles from the agglutinated particles generated by the agglutination of multiple carrier particles by the scattered light intensity of respective particles and count the single particle number (M) and the agglutinated particle number (P) ; (c) obtain the total particle number (T) which is the sum of M and P to calculate P/T, which is the agglutination rate.

Since a reaction can be caught at a stage where two carrier particles agglutinate, an extremely highly sensitive immunoassay becomes possible in this method. It is possible to use various methods for the measurement of this agglutination level depending on the measuring concentration range of the measuring object substance (e.g., a method measuring particle levels agglutinated at a fixed number or more).

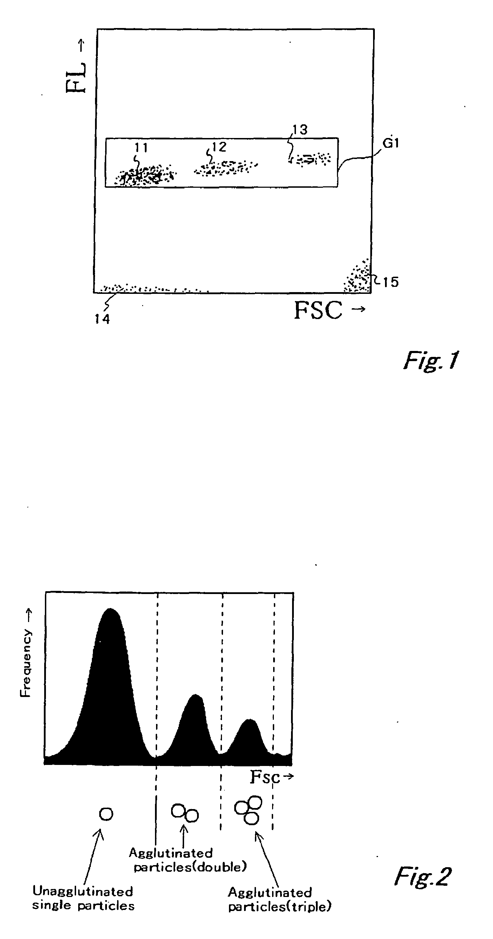

One example of a scattergram which is a measuring example in accordance with the present invention is shown in Fig. 1. Latex particles containing fluorescent dye as the carrier particles were blended with a blood specimen to render a sample liquid. Using a flow cytometer, the fluorescent intensity and the forward scattered light intensity were detected as the optical information from the sample liquid. With the detected optical information, the scattergram in Fig. 1 was obtained using fluorescent intensity as the vertical axis and forward scattered light intensity as the horizontal axis. The carrier particles differ in forward scattered light intensity depending on patterns of agglutination such as non-agglutinated single particles 11, agglutinated double particles 12 resulting from the agglutination of two particles, and agglutinated triple particles 13 resulting from the agglutination of three carrier particles. The respective carrier particles emerge as segregated populations on the scattergram. Chylomicrons 14 and erythrocytes 15 emerge as particles other than the measuring object substances in the specimen; however, both are low in fluorescent intensity and can be differentiated from carrier particles.

A region is set on the scattergram to surround the respective carrier particles. An example of the particle size distribution of the carrier particles emerging within the fixed region G1 is shown in the histogram in Fig. 2. The vertical axis denotes the number of particles (frequency) and the horizontal axis denotes the forward scattered light intensity. The agglutinated particles and single particles can be differentiated by setting a threshold for the forward scattered light intensity.

In accordance with the present invention, the first and second carrier particles are differentiated on the basis of the first optical information. The fluorescent dye, dystuff, luminescent substrate or the like is contained in the first and second carrier particles, and it is based on such that the first and second carrier particles are differentiated by appropriately selecting type and content level. The first and second carrier particles can be differentiated by the use of fluorescence, absorbance, phosphorescence, chemiluminescence, biological luminescence as the first optical information.

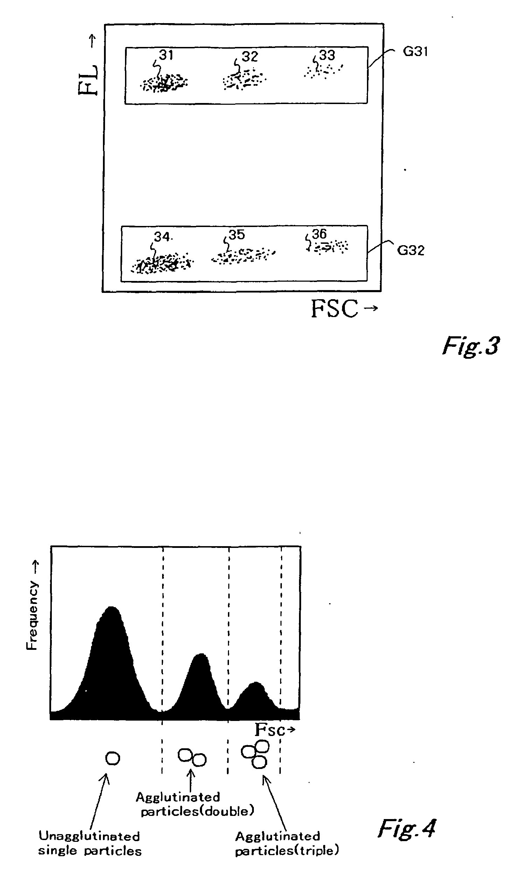

An example of a scattergram which is a measuring example in accordance with the present invention is shown in Fig. 3. Two types of latex particles containing fluorescent dye at different concentrations were used, which rendered the first and second carrier particles, respectively. The first and second carrier particles were simultaneously reacted with a specimen, which rendered a sample liquid, and the fluorescent intensity and the forward scattered light intensity were detected as the optical information by a flow cytometer. With the optical information detected, the scattergram in Fig. 3 was obtained using fluorescent intensity as the vertical axis and forward scattered light intensity as the horizontal axis.

The first carrier particles (31, 32 and 33) and the second carrier particles (34, 35 and 36) exhibit different levels of fluorescent intensity, and emerge at different locations on the scattergram due to the difference in concentrations of the fluorescent dye contained. Thus, it is possible to differentiate the first and second carrier particles on the scattergram. Moreover, the respective carrier particles (e.g., non-agglutinated single particles, double particles resulting from the agglutination of two carrier particles and triple carrier particles resulting from the agglutination of three particles) emerge at different locations as respective populations on the scattergram depending on the difference in the forward scattered light intensity. In the drawing, the single particles 31 of the first carrier particles, the agglutinated double particles 32 of the first carrier particles and the agglutinated triple particles 33 of the first carrier particles emerge into respective segregated populations due to the difference in forward scattered light intensity. Likewise, the single particles 34 of the second carrier particles, the agglutinated double particles 35 of the second carrier particles and the agglutinated triple particles 36 of the second carrier particles also emerge into respective segregated populations due to the difference in forward scattered light intensity.

In this case, regions are fixed on the scattergram to surround the first and second carrier particles, respectively. A histogram is made which represents the particle size distribution of the carrier particle emerging within each region. Fig. 4 shows one example of a histogram made on the basis of forward scattered light intensity emerging within the region G31 of the first carrier particles. The vertical axis denotes frequency (particle number) and the horizontal axis denotes forward scattered light intensity. The agglutinated particles and single particles can be differentiated by setting a threshold for the forward scattered light intensity.

The following examples illustrate features in accordance with the present invention, and are provided solely by way of illustration. They are not intended to limit the scope of the appended claims or their equivalents.

### Examples

Latex particles in which a prescribed concentration of fluorescent dye capable of being excited by laser light is contained (hereinafter, referred to as "fluorescent latex particles") were used as carrier particles. The carrier particles are immobilized with an antibody (or antigen) which responds to an antigen (or antibody) which is a detection object substance to render a fluorescent latex reagent. A specimen containing an antigen (or antibody) which is a measuring object substance, the fluorescent latex reagent and a reaction buffer were mixed to prepare a sample liquid, and the forward scattered light and the fluorescence were detected from the sample liquid. The measuring object substance in the specimen was detected by making a two dimensional scattergram in which the detected forward scattered light signal and fluorescent signal were rendered parameters, and analyzed.

### 1. Apparatus

A commercially available flow cytometer, FACSCalibur (supplied by Becton Dickinson) and an automatic immunoassay apparatus manufactured by the present inventors were used in the examples herein. In using a FACSCalibur, it is necessary to prepare the sample liquid by mixing and incubating the specimen and reagents manually beforehand. When the prepared sample liquid is set and a measuring operation is performed, the apparatus detects the forward scattered light and fluorescence and carries out the creation of the two dimensional scattergram. On the other hand, the automatic immunoassay apparatus made by the present inventors functions to prepare the sample liquid automatically and automatically carries out acts ranging from the preparation of the sample liquid and the detection of the forward scattered light and fluorescence to the creation and analysis of a two dimensional scattergram and analysis.

The automatic immunoassay apparatus manufactured by the present inventor is illustrated. This apparatus simultaneously carries out four parameters of immunoassays for one specimen, and the fluorescent latex reagent used on the apparatus includes four types of fluorescent latex particles depending on the measuring parameters. The four types of fluorescent latex particles contain the fluorescent dye at different concentrations from one another depending on the types, and are immobilized with antibodies (or antigens) responding to the measuring parameters depending on the types. Details of this fluorescent latex reagent are described below.

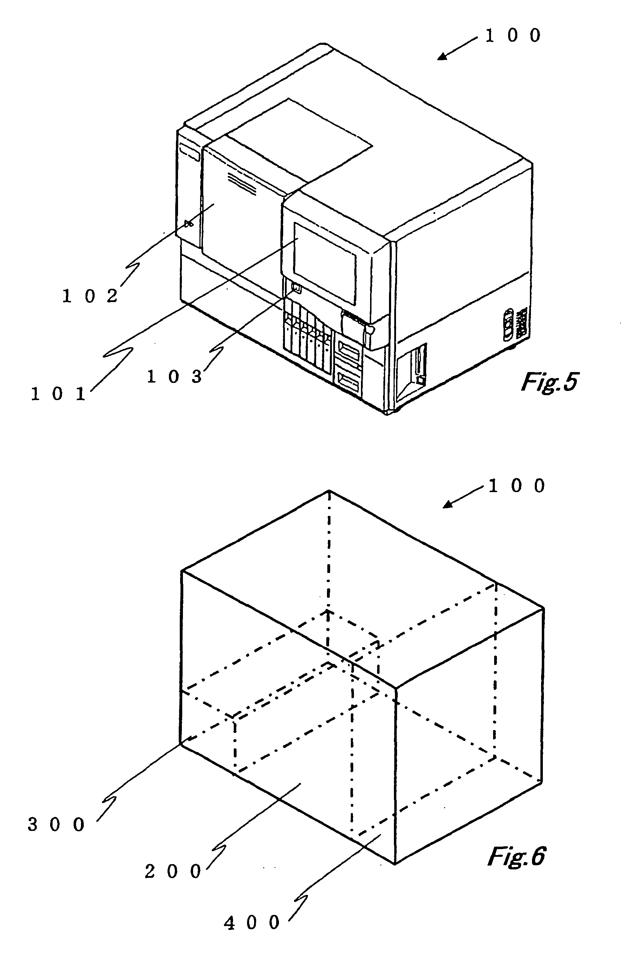

Fig. 5 shows the surface appearance of an automatic immunoassay apparatus 100. A front surface includes a liquid crystal touch panel 101 for inputting various settings and displays outputting measuring results, a cover 102 which covers a sample preparing portion 200 described below, and a start switch 103.

Fig. 6 shows an internal configuration of the automatic immunoassay apparatus 100. A control portion 400 which conducts operation of the apparatus and analysis processing is included in a space at the right side of the apparatus. A light detecting portion 300 for detecting signals from the sample liquid is included at a left lower space of the apparatus. In addition, the remaining space includes the sample preparing portion 200 for preparing the sample liquid.

The respective portions of the sample preparing portion 200, the light detecting portion 300, and the control portion 400 are illustrated below.

### Configuration of the sample preparing portion 200

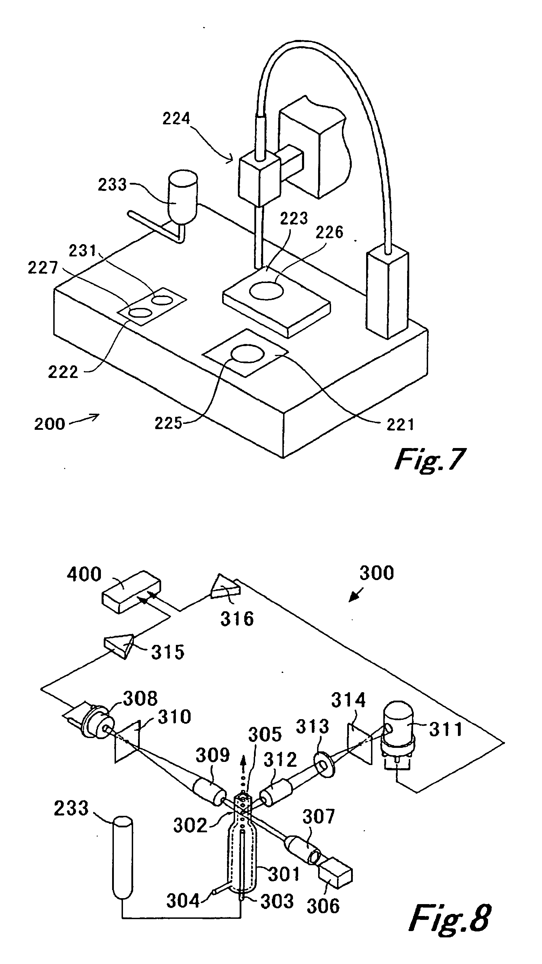

Fig. 7 is an illustration showing the sample preparing portion 200. It includes a specimen setting portion 221 at the front right side, a reagent setting portion 222 at the near left side, and an incubator 223 at the back side. In addition, it includes a dispensing device 224, which is movable up and down as well as from side to side. It is configured such that by opening the cover 102 in Fig. 6, the sample preparing portion 200 shown in Fig. 7 appears, and an operator sets various vessels as described below. It is configured such that a specimen vessel 225 in which the specimen is placed is set at the specimen setting portion 221, and a reaction vessel 226 is set at the incubator 223. In addition, it is configured such that a reagent vessel 227 in which the fluorescent latex reagent is placed and a reagent vessel 231 in which the reaction buffer is placed are set at the reagent setting portion 222. The incubator 223 is configured such that a liquid in the set reaction vessel 226 is shaken and stirred while being retained at a prescribed temperature. The dispensing device 224 aspirates and discharges a prescribed amount of liquid from its front end, and is configured to be movable up and down as well as from side to side by a drive unit not shown in the drawing. The sample vessel 233 is connected with a flow cell 301 of the light detecting portion 300 described below.

### Configuration of the light detecting portion 300

Fig. 8 is an illustration showing the configuration of the light detecting portion 300. The light detecting portion 300 has a flow cell 301 to flow the sample liquid. The flow cell 301 has an orifice portion 302 which is a portion where laser light is irradiated and an inner flow path is thinly narrowed down, a nozzle 303 which emits a jet of the sample liquid upward to the orifice portion, a supply port 304 of a sheath liquid, and a waste liquid port 305. In addition, the light detecting portion 300 has a laser light source 306 for irradiation of laser light (it is a red semiconductor laser light source which emits laser light at a wavelength of 635 nm), a condenser lens 307 which condenses the laser light irradiated from the laser light source to a sheath flow cell, a photodiode 308 which receives the forward scattered light from the particles and converts to electric signals, a collector lens 309 and a pin hole 310 to condense the forward scattered light to the photodiode, a photo multiplier tube 311 which receives the fluorescence and converts to electric signals, a collector lens 312, a filter 313, a pin hole 314 to condense the fluorescence to the photo multiplier tube, and amplifiers 315 and 316 which amplify the electric signals output from the photodiode 308 and the photo multiplier tube 311 and output to the control portion 400.

### Configuration of the control portion 400

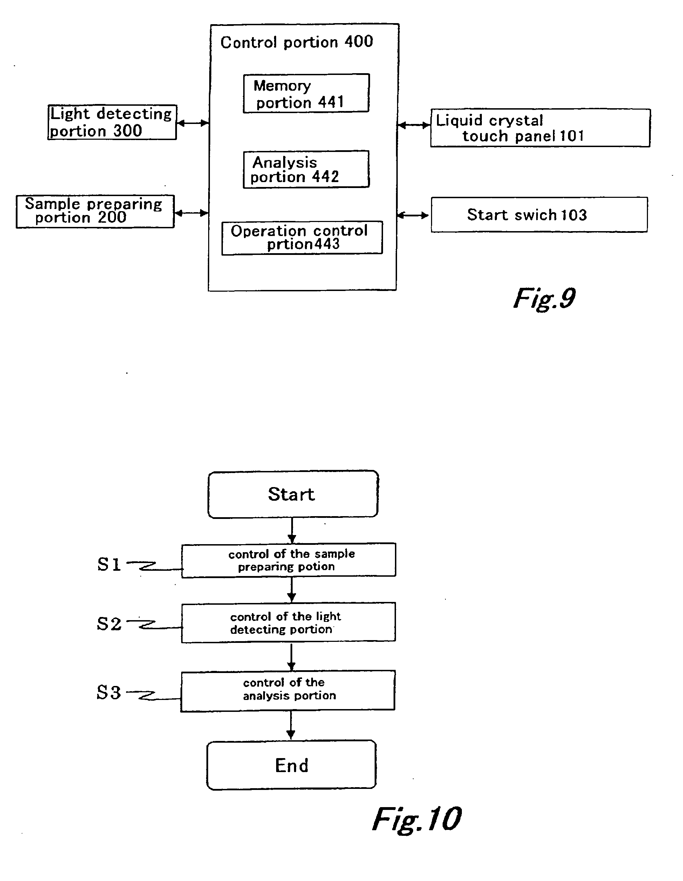

Fig. 9 is a block diagram showing the configuration of the control portion 400 and the relationship of the control portion 400 to respective portions of the apparatus. The control portion 400 has a microcomputer having a central processing unit (CPU) and memory devices such as ROM and RAM circuits which process signals sent from the light detecting portion 300, etc. The control portion 400 carries out functions such as a memory portion 441, an analysis portion 442, and an operation control portion 443. The memory portion 441 memorizes analysis programs for the analysis of signals obtained from the particles in the sample and control programs to control respective portions of the apparatus. In addition, it stores signal data detected at the light detecting portion 300 and results processed by the analysis programs. The analysis portion 442 processes the signals detected at the light detecting portion 300 by measuring the sample liquid and analyzes the signals after the processing. The analyzed results at the analysis portion 442 are output on the liquid crystal touch panel 101. The operation control portion 443 controls the operations at respective portions of the apparatus on the basis of the control programs memorized at the memory portion 441.

### Operations of the apparatus

Fig. 10 is a flowchart showing the entire controls of the automatic immunoassay apparatus 100 by the control programs. When the operator pushes a starting switch 103, the control programs are started up, and S1 (control of the sample preparing portion), S2 (control of the light detecting portion), and S3 (control of the analysis portion) are sequentially performed. This controls the sample preparing portion 200, the light detecting portion 300 and the analysis portion 442, and a series of operations of the automatic immunoassay apparatus 100 is automatically performed. The operation of each portion of the apparatus by the S1, S2 and S3 is illustrated below.

### S1 (control of the sample preparing portion)

The operation of the sample preparing portion 200 by control of the sample preparing portion is illustrated using Fig. 7. First, the dispensing device 224 aspirates 80 µl of a reaction buffer from the sample vessel 231 at the sample setting portion 222, and next aspirates 10 µl of a specimen from the specimen vessel 225 set at the specimen setting portion 221. The reaction buffer and the specimen aspirated are dispensed into the reaction vessel 226 set at the incubator 223. Next, the dispensing device 224 aspirates 10 µl of a fluorescent latex reagent from the reagent vessel 227 at the reagent setting portion 222, and dispenses it into the reaction vessel 226. Subsequently, the incubator 223 incubates the reaction vessel 226 in which the specimen, fluorescent latex reagent and reaction buffer are placed by shaking/stirring for 15 min while maintaining the temperature at 45°C to prepare a sample liquid. The dispensing device 224 aspirates the sample liquid after the incubation, and dispenses it into the sample vessel 233. A dilution liquid is placed in the sample vessel 233 beforehand, and the dispensed sample liquid is diluted 51-fold. The sample liquid diluted in the sample vessel 233 is flowed to the flow cell 301 at the light detecting portion 300.

### S2 (control of the light detecting portion)

The operation of the light detecting portion 300 by the control of the light detecting portion is illustrated in Fig. 8. As described above, when the sample liquid is supplied into the sample vessel 233, the sample liquid is led to the nozzle 303 by the operation of pumps and valves not shown in the drawing. The sample liquid is discharged from the nozzle 303 to the flow cell 301. Simultaneously, a sheath liquid is supplied from a sheath liquid vessel not shown in the drawing to the flow cell 301 via the sheath supplying port 304. In such a way, the sample liquid is contained with the sheath liquid in the flow cell 301 and flows through the thinly narrowed orifice portion 302. Narrowing down a flow of the sample liquid to a size approximately equivalent to the particle diameter can flow the particles contained in the sample liquid in a line through the orifice portion 302. The laser light emitted from the laser light source 306 and narrowed down at the condenser lens 307 is irradiated to the sample liquid which flows at the orifice portion 302. The forward scattered light emitted from the particle in the sample liquid received by the laser light is collected by the collector lens 309. The forward scattered light which has passed through the pin hole 310 is received at the photodiode 308 and photo-electrically transferred to become a forward scattered light signal. The fluorescence emitted from the particle in the sample liquid given the laser light is collected by the contact lens 312. The fluorescence which has passed through the filter 313 and the pin hole 314 is received at the photo multiplier tube 311, and photo-electrically transferred to a fluorescence signal. The forward scattered light signal and the fluorescence signal are amplified at the amplifiers 315 and 316, respectively, subsequently sent to the control portion 400, and stored at the memory portion 441 as data for each particle.

### S3 (control of the analysis portion)

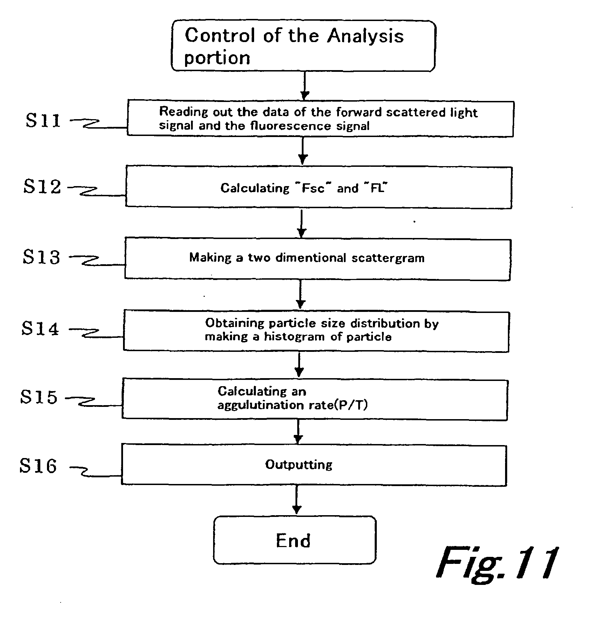

Details of the analysis portion control are illustrated in the flowchart of Fig. 11. Respective stages in the flowchart are as follows.

S11: The data of the forward scattered light signal and the fluorescence signal is read from the memory portion.

S12: A forward scattered light intensity (Fsc) and a fluorescence intensity (FL) are calculated for each particle on the bases of peak levels of the forward scattered light signal and the fluorescence signal, respectively.

S13: A two dimensional scattergram is made using Fsc and FL for each particle calculated at S12 as the parameters. Regions in which four types of fluorescent latex particles responding to the measuring parameters are forecasted to emerge have been predetermined on this scattergram.

S14: A histogram of particles which have emerged in respective regions predetermined on the two dimensional scattergram is made to obtain particle size distribution.

S15: The numbers of the single particles (M) and agglutinated particles (P) in respective regions, and the sum of these (T) are obtained, and a agglutination rate (P/T) of the particles which have emerged in each region is calculated.

S16: The two dimensional scattergram, the histogram of the respective regions made above, the agglutinated rate, and the P/T calculated above are output and displayed on the liquid crystal touch panel 101.

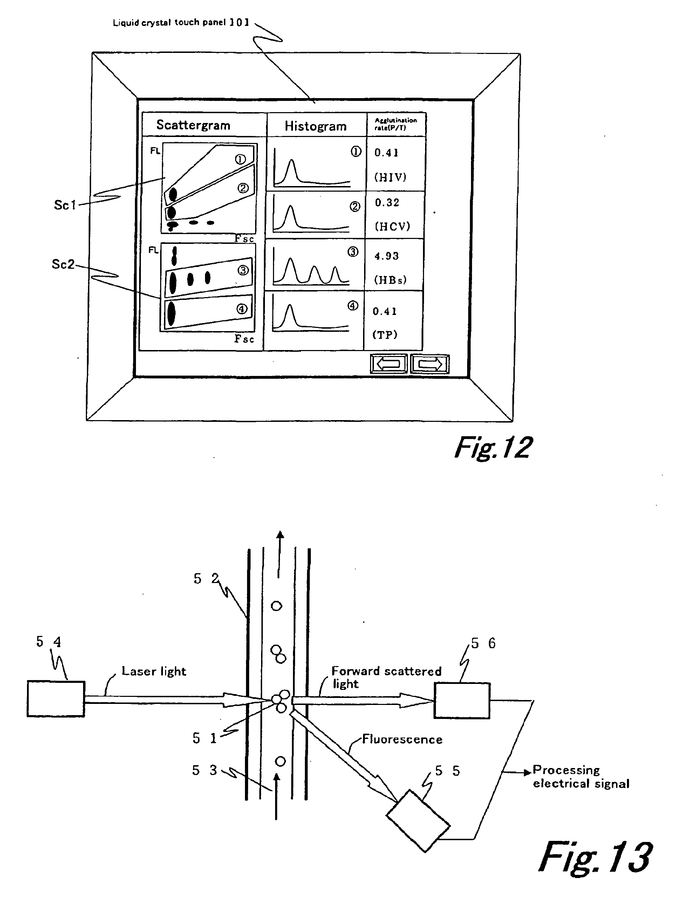

Fig. 12 is a drawing showing a screen including the scattergrams made at S13, the histograms made at S14, and the agglutination rates (P/T) calculated at S15 are output on the liquid crystal touch panel at S16. On this screen, the two dimensional scattergrams, Sc1 and Sc2 are displayed at upper and lower sides. These correspond to the where the measuring results obtained from the same specimen are output by changing the display patterns. For both, the horizontal axis denotes Fsc and the vertical axis denotes F1. However, since the values (F1) on the vertical axis are log-transformed in the scattergram displayed at the lower side, lower value parts of FL are relatively magnified and displayed compared to higher value parts. Thus, among four types of the fluorescent latex particles contained in the fluorescent latex reagent, for two types of the fluorescent latex particles where the concentration of the fluorescent dye contained is higher, the regions where the particles emerge are set and displayed on the two dimensional scattergram, such that Sc1 is displayed at the upper side (region 1, region 2). In addition, for two types of the fluorescent latex particles where the concentration of the fluorescent dye contained is lower, the regions where the particles emerge are determined and displayed on the two dimensional scattergram, such that Sc2 is displayed at the lower side (region 3, region 4). Region 1 is a region where the fluorescent latex particles in which the concentration of fluorescent dye is the highest of the four types emerge. Region 2 is a region where the fluorescent latex particles in which the concentration of fluorescent dye is the second highest of the four types emerge. Region 3 is a region where the fluorescent latex particles in which the concentration of fluorescent dye is the third highest of the four types emerge. Region 4 is a region where the fluorescent latex particles in which the concentration of fluorescent dye is the lowest of the four types emerge. In addition, the histograms 1 through 4 and the agglutination rates corresponding to the regions 1 through 4 are displayed on the screen.

### 2. Preparation of reagents

The reagents used for the immunoassays in FACSCalibur and the automatic immunoassay apparatus including the fluorescent latex reagent, reaction buffer and dilution liquid are described below.

### Fluorescent latex reagent

A fluorescent latex particle which makes a latex particle in which the particle diameter is 0.78 µm and the surface is sulfate containing a red fluorescent dye (capable of being excited with laser light at a wavelength of 640 nm) at a prescribed concentration was used as a carrier particle. The carrier particle is immobilized with an antibody (or an antigen) which responds to an antigen (or an antibody) which is a detecting object substance. An anti-HBs antibody, TP antigen, anti-CEA antibody, anti-AFP antibody, HCV antigen, HIV antigen, anti-FRN antibody, anti-PSA antibody or the like is immobilized by hydrophobic bonds to different fluorescent latex particles, respectively. They are used alone or in combination by being mixed in one liquid to prepare various fluorescent latex reagents. The preparation of each fluorescent latex reagent is carried out as in (1)-(8) described below.

### (1) Preparation of the fluorescent latex reagent for detection of HBs antigen

The fluorescent latex reagent for detection of the HBs antigen was prepared by immobilizing the anti-HBs antibody to the fluorescent latex particles of a fluorescent dye concentration of 1% (w/v). First, 50 µl of 10% fluorescent latex particle suspension (w/v) was added to 950 µl of GTA buffer (0.53 mg/mL of 3,3-dimethyl glutaric acid, 0.4 mg/mL of Tris, 0.35 mg/mL of 2-amino-2-methyl-1,3-propanediol, pH 4.6) containing 60 µg of anti-HBs antibody (mouse monoclonal antibody, commercially available product), and kept at 20°C for 2 hours. This was centrifuged at 10000 ×g for 10 min, a supernatant was discarded, 1 mL of GTA buffer containing 1% (w/v) bovine serum albumin (commercially available product) was added to a pellet, and sonicated to disperse. The steps from the centrifuging through the dispersing were repeated several times. Finally, after centrifuging and discarding the supernatant, 1 mL of GTA buffer (pH 6.2)containing 220 mg/mL of glycerine and 0.3% (w/v) bovine serum albumin was added to the pellet, and sonicated to disperse. This was rendered as the fluorescent latex reagent for detection of the the HBs antigen.

### (2) Preparation of the fluorescent latex reagent for detection of anti-TP antibody

The fluorescent latex reagent for detection of the anti-TP antibody was prepared by immobilizing the TP antigen to the fluorescent latex particles of a fluorescent dye concentration of 0.1% (w/v). First, 50 µl of 10% the fluorescent latex particles suspension (w/v) was added to 950 µl of 10 mM PBS (pH 4.0) containing 50 µg of TP antigen (commercially available product), and kept at 4°C for 24 hours. This was centrifuged at 10000 ×g for 10 min, a supernatant was discarded, 1 mL of 0.1 M PBS buffer (pH 7.0) containing 1mg/mL of bovine serum albumin was added to a pellet, and sonicated to disperse. The steps from the centrifuging through the dispersing were repeated several times. Finally, after centrifuging and discarding the supernatant, 1 mL of PBS buffer (pH 7.0) containing 1 mg/mL of bovine serum albumin was added, and sonicated to disperse. This was rendered as the fluorescent latex reagent for detection of the the anti-TP antibody.

### (3) Preparation of the fluorescent latex reagent for detection of AFP antigen

The fluorescent latex reagent for detection of the AFP antigen was prepared by immobilizing the anti-AFP antibody to the fluorescent latex particles of a fluorescent dye concentration of 0.01% (w/v). First, 50 µl of 10% fluorescent latex particles suspension (w/v) was added to 950 µl of GTA buffer (0.53 mg/mL of 3,3-dimethyl glutaric acid, 0.4 mg/mL of Tris, 0.35 mg/mL of 2-amino-2-methyl-1,3-propanediol, pH 4.6) containing 60 µg of anti-CEA antibody (goat polyclonal antibody, commercially available product), and kept at 20°C for 2 hours. This was centrifuged at 10000 ×g for 10 min, a supernatant was discarded, 1 mL of GTA buffer containing 1% (w/v) bovine serum albumin (commercially available product) was added to a pellet, and sonicated to disperse. The steps from the centrifuging through the dispersing were repeated several times. Finally, after centrifuging and discarding the supernatant, 1 mL of GTA buffer (pH 6.2) containing 220 mg/mL of glycerine and 0.3% (w/v) bovine serum albumin was added to the pellet, and sonicated to disperse. This was rendered as the fluorescent latex reagent for detection of the AFP antigen.

### (4) Preparation of the fluorescent latex reagent for detection of the CEA antigen

The fluorescent latex reagent for detection of the CEA antigen was prepared by immobilizing the anti-CEA antibody to the fluorescent latex particles of a fluorescent dye concentration of 0.001% (w/v). First, 50 µl of 10% fluorescent latex particles suspension (w/v) was added to 950 µl of GTA buffer (0.53 mg/mL of 3,3-dimethyl glutaric acid, 0.4 mg/mL of Tris, 0.35 mg/mL of 2-amino-2-methyl-1,3-propanediol, pH 4. 6) containing 60 µg of the anti-CEA antibody (rabbit polyclonal antibody, commercially available product), and kept at 20°C for 2 hours. This was centrifuged at 10000 ×g for 10 min, a supernatant was discarded, 1 mL of GTA buffer containing 1% (w/v) bovine serum albumin (commercially available product) was added to a pellet, and sonicated to disperse. The steps from the centrifuging through the dispersing were repeated several times. Finally, after centrifuging and discarding the supernatant, 1 mL of GTA buffer (pH 6.2) containing 220 mg/mL of glycerine and 0.3% (w/v) bovine serum albumin was added to the pellet, and sonicated to disperse. This was rendered as the fluorescent latex reagent for detection of the CEA antigen.

### (5) Preparation of the reagent for simultaneous measurement of infection marker-four parameters

The fluorescent latex particles for detection of the HBs antigen, the fluorescent latex particles for detection of the anti-TP antibody, the fluorescent latex particles for detection of the anti-HCV antibody and the fluorescent latex particles for detection of the anti-HIV antibody were prepared, and mixed in one liquid to prepare the fluorescent latex reagent for simultaneously detecting the HBs antigen, anti-TP antibody, anti-HCV antibody and anti-HIV antibody.

As the fluorescent latex reagent for detection of the HBs antigen and the fluorescent latex reagent for detection of the anti-TP antibody, those prepared in (1) and (2) above were used.

The fluorescent latex particles for detection of the anti-HCV antibody was prepared by immobilizing the HCV antigen to the fluorescent latex particles of a fluorescent dye concentration of 3% (w/v). First, 50 µl of 10% of the fluorescent latex particles suspension (w/v) was added to 950 µl of 10 mM glycine buffer (pH 2.0) containing 8 types of complex antigens, i.e., two types of recombinant antigens corresponding to viral protein derived from NS3 of the HCV non-structural region, two synthetic peptide antigens corresponding to viral protein derived from NS4 inclusion, and one type of synthetic peptide antigen corresponding to viral protein derived from NS5, as well as 3 types of synthetic peptide antigens corresponding to viral protein derived from the core of the structural region, and was kept at 37°C for one hour. This was centrifuged at 10000 ×g for 10 min, a supernatant was discarded, 1 mL of 1.2% (w/v) Tris buffer (pH 8.0) containing 2% bovine serum albumin (commercially available product) was added to a pellet, and sonicated to disperse, and incubated at 45°C for 2 hours to perform blocking. The steps from the centrifuging through the dispersing were repeated several times. Finally, after centrifuging and discarding the supernatant, 1 mL of 1.2% (w/v) Tris buffer (pH 8.0) containing 220 mg/mL of glycerine and 0.3% (w/v) bovine serum albumin was added to the pellet, and sonicated to disperse the fluorescent latex particles for detection of the anti-HCV antibody in a liquid.

The fluorescent latex particles for detection of the anti-HIV antibody was prepared by immobilizing the HIV antigen to the fluorescent latex particles of a fluorescent dye concentration of 5% (w/v). First, 50 µl of 10% of the fluorescent latex particles suspension (w/v) was added to 950 µl of 10 mM glycine buffer (pH 2.0) containing HIV-1 gag (group specific antigen) region antigen, HIV-1 env (envelope) region antigen, HIV-1 pol (polymerase) region antigen and HIV-2 env antigen, and was kept at 37°C for one hour. This was centrifuged at 10000 ×g for 10 min, a supernatant was discarded, 1 mL of 1.2% (w/v) Tris buffer (pH 8.0) containing 2% bovine serum albumin (commercially available product) was added to a pellet, and sonicated to disperse, and incubated at 45°C for 2 hours to perform blocking. The steps from the centrifuging through the dispersing were repeated several times. Finally, after centrifuging and discarding the supernatant, 1 mL of 1.2% (w/v) Tris buffer (pH 8.0) containing 220 mg/mL of glycerine and 0.3% (w/v) bovine serum albumin was added to the pellet, and sonicated to disperse the fluorescent latex particles for detection of the anti-HIV antibody in a liquid.

Equivalent amounts of the liquid above in which the fluorescent latex reagent for detection of the anti-HCV antibody were dispersed, the liquid above in which the fluorescent latex particles for detection of the anti-HIV antibody were dispersed, the fluorescent latex reagent above for detection of the HBs antigen, and the fluorescent latex reagent above for detection of the anti-TP antibody were mixed to render the fluorescent latex reagent for the simultaneous measurement of the infection marker four parameters.

### (6) Preparation of the reagent for simultaneous measurement of tumor marker-four parameters

The fluorescent latex reagent for simultaneously detecting the FRN antigen, CEA antigen, AFP antigen and PSA antigen was prepared by preparing the fluorescent latex particles for detection of the FRN antigen, the fluorescent latex particles for detection of the CEA antigen, the fluorescent latex particles for detection of the AFP antigen and the fluorescent latex particles for detection of the PSA antigen, and mixing them in one liquid.

The fluorescent latex particles for detection of the FRN antigen were prepared by immobilizing the anti-FRN antibody to the fluorescent latex particles of a fluorescent dye concentration of 0.1% (w/v). First, 50 µl of 10% of the fluorescent latex particles suspension (w/v) was added to 950 µl of GTA buffer (0.53 mg/mL of 3,3-dimethyl glutaric acid, 0.4 mg/mL of Tris, 0.35 mg/mL of 2-amino-2-methyl-1, 3-propanediol, pH 4.6) containing 50 µg of anti-FRN antibody (goat polyclonal antibody, commercially available product), and kept at 20°C for 2 hours. This was centrifuged at 10000 ×g for 10 min, a supernatant was discarded, 1 mL of GTA buffer containing 1% (w/v) bovine serum albumin (commercially available product) was added to a pellet, and sonicated to disperse. The steps from the centrifuging through the dispersing were repeated several times. Finally, after centrifuging and discarding the supernatant, 1 mL of GTA buffer (pH 6.2) containing 220 mg/mL of glycerine and 0.3% (w/v) bovine serum albumin was added to the pellet, and sonicated to disperse the fluorescent latex particles for detection of the FRN antigen in a liquid.

The fluorescent latex particles for detection of the CEA antigen were prepared by immobilizing the anti-CEA antibody to the fluorescent latex particles of a fluorescent dye concentration of 1% (w/v) by a similar procedure as that used in the preparation of the fluorescent latex reagent for detection of the CEA antigen in (4) above.

The fluorescent latex particles for detection of the AFP antigen were prepared by immobilizing the anti-AFP antibody to the fluorescent latex particles of fluorescent dye concentration of 3% (w/v) by a similar procedure as that used in the preparation of the fluorescent latex reagent for detection of the AFP antigen in (3) above.

The fluorescent latex reagent for detection of the PSA antigen was prepared by immobilizing the anti-PSA antibody to the fluorescent latex particles of a fluorescent dye concentration of 5% (w/v). First, 50 µl of 10% fluorescent latex particle suspension (w/v) was added to 950 µl of GTA buffer (0.53 mg/mL of 3,3-dimethyl glutaric acid, 0.4 mg/mL of Tris, 0.35 mg/mL of 2-amino-2-methyl-1,3-propanediol, pH 4.6) containing 60 µg of anti-PSA antibody (rabbit polyclonal antibody, commercially available product), and kept at 20°C for 2 hours. This was centrifuged at 10000 ×g for 10 min, a supernatant was discarded, 1 mL of GTA buffer containing 1% (w/v) bovine serum albumin (commercially available product) was added to a pellet, and sonicated to disperse. The steps from the centrifuging through the dispersing were repeated several times. Finally, after centrifuging and discarding the supernatant, 1 mL of GTA buffer (pH 6.2) containing 220 mg/mL of glycerine and 0.3% (w/v) bovine serum albumin was added to the pellet, and sonicated to disperse the fluorescent latex particles for detection of the PSA antigen in a liquid.

Equivalent amounts of the liquids prepared above in which the fluorescent latex particles for detection of the FRN antigen, the fluorescent latex particles for detection of the CEA antigen, the fluorescent latex particles for detection of the AFP antigen and the fluorescent latex particles for detection of the PSA antigen were dispersed are mixed to render the fluorescent latex reagent for the simultaneous detection of the tumor marker-four parameters.

### Reaction buffer

The reaction buffer was prepared as follows: 1.6 mg/mL of 3,3-dimethyl glutaric acid, 1.1 mg/mL of 2-amino-2-methyl-1,3-propanediol, 18.18 mg/mL of Tris, 5% (w/v) of bovine serum albumin, 0.6% (w/v) of dextran (commercially available product), 15% (w/v) of heat-treated bovine serum albumin, 40% (w/v) of pepsin-treated bovine serum albumin, 0.4% (w/v) of PVA (commercially available product), 0.15% (w/v) of sodium chloride and 0.045% (w/v) sodium azide, pH 6.0. The reaction buffer can be added along with the fluorescent latex reagent to the specimen which becomes the measuring object.

### Dilution liquid

In the automatic immunoassay apparatus 100, a prescribed amount of dilution liquid is placed in the sample vessel 233 prior to the measurement. The sample liquid prepared by blending and incubating the specimen with the fluorescent latex reagent and the reaction buffer as described above is diluted to a prescribed concentration by being placed in the sample vessel 233. A clean sheath (Sysmex Corporation) was used for this dilution liquid.

### 3. Single parameter measurement

The results of the assay by FACSCalibur using the fluorescent latex reagent supporting a single measurement parameter are shown. The specimens as the measuring objects were prepared as follows.

### Specimens

Human normal whole blood and human normal whole blood to which HBs antigens at six scales by concentrations were added were prepared to render the specimen series containing the HBs antigen (HBs 1 through HBs 7). The concentration of HBs antigen in each specimen in the specimen series containing HBs antigen is as follows:
HBs 1: 0 U/mL, HBs 2: 1 U/mL, HBs 3: 3 U/mL, HBs 4: 9 U/mL, HBs 5: 27 U/mL, HBs 6: 81 U/mL, and HBs 7: 243 U/mL.

In addition, human normal whole blood and human normal whole blood to which anti-TP antibodies at six scales by concentration were added were prepared to render the specimen series containing anti-TP antibody (TP 1 through TP 7). The concentration of the anti-TP antibody in each specimen in the specimen series containing the anti-TP antibody is as follows:
TP 1: 0 mIU, TP 2: 16.5 mIU, TP 3: 41.2 mIU, TP 4: 103.1 mIU, TP 5: 257.6 mIU, TP 6: 644 mIU, and TP 7: 1610 mIU.

In addition, human normal whole blood and human normal whole blood to which AFP antigens at six scales by concentration were added were prepared to render the specimen series containing the AFP antigen (AFP 1 through AFP 7). The concentration of the AFP antigen in each specimen in the specimen series containing the AFP antigen is as follows:
AFP 1: 0 ng/mL, AFP 2: 2 ng/mL, AFP 3: 8 ng/mL, AFP 4: 32 ng/mL, AFP 5: 128 ng/mL, AFP 6: 512 ng/mL, and AFP 7: 2048 ng/mL.

In addition, human normal whole blood and human normal whole blood to which CEA antigens at six scales by concentration were added were prepared to render the specimen series containing the CEA antigen (CEA 1 to CEA 7). The concentration of the CEA antigen in each specimen in the specimen series containing the CEA antigen is as follows:
CEA 1: 0 ng/mL, CEA 2: 0.5 ng/mL, CEA 3: 2 ng/mL, CEA 4: 8 ng/mL, CEA 5: 32 ng/mL, CEA 6: 128 ng/mL, and CEA 7: 512 ng/mL.

In addition, the specimen S1 in which four types of antigens or an antibody (i.e., HBs antigen, anti-TP antibody, AFP antigen and CEA antigen) was prepared in human normal whole blood such that the concentration was the same as that in 4 of the specimen series above (i.e., the specimen S1 contains 9 U/mL of HBs antigen, 103.1 mIU of anti-TP antibody, 32 ng/mL of AFP antigen and 8 ng/mL of CEA antigen). The specimen S2 was prepared such that the concentration was the same as that in 7 (i.e., the specimen S2 contains 243 U/mL of HBs antigen, 1610 mIU of anti-TP antibody, 2048 ng/mL of AFP antigen and 512 ng/mL of CEA antigen).

### Measurement

To each of the 28 specimens (each 40 µl) prepared above was added 10 µl of the fluorescent latex reagent responding to the measuring object substance contained in each specimen series and 350 µl of the reaction buffer. The resultant mixture was incubated at 45°C for 15 min and then diluted with 10 mM PBS buffer 51-fold to prepare a total of 28 samples for measurement. Each sample liquid prepared was applied to FACSCalibur and measured.

FACSCalibur is a flow cytometer having the function of irradiating a laser at a wavelength of 635 nm from the red semiconductor laser light source and detecting the forward scattered light signal and the side fluorescence signal from the sample containing the fluorescent latex reagent. Fig. 13 is a schematic view showing a detection system of FACSCalibur. The sample containing the carrier particles 51 is led to a flow cell 52, and forms sample flow 53. The laser light emitted from the red semiconductor laser light source 54 irradiates the sample flow 53 in the flow cell 52. The fluorescence and the forward scattered light which occur when the carrier particle 51 passes across the irradiation region of the laser light are received at a photo multiplier tube 55 and a photodiode 56, respectively, and photo-electrically transformed to become electric signals, which are then processed and analyzed. In this way, the fluorescence signal and the forward scattered light signal are detected and processed into electrical signals.

### Analysis

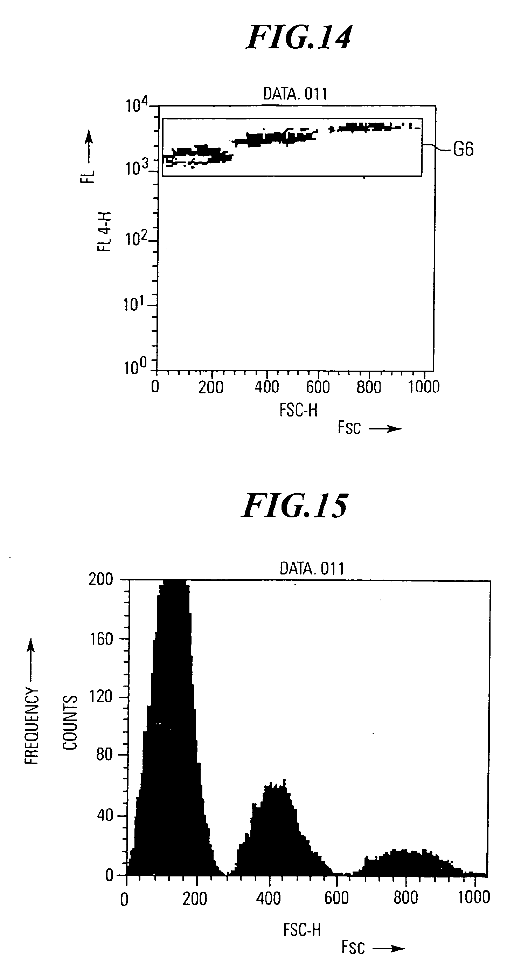

A two dimensional scattergram was manufactured using the forward scattered light signals and fluorescence signals detected from each sample as the parameters. Fig. 14 is a two dimensional scattergram obtained by measuring the sample in which the fluorescent latex reagent for detection of the HBs antigen was added to HBs 4 which was a specimen containing the HBs antigen. The vertical axis denotes the fluorescent intensity and the horizontal axis denotes the forward scattered light intensity. Due to the difference in the forward scattered light intensity, non-agglutinated single particles, agglutinated double particles, and agglutinated triple particles emerge into respective segregated populations on the scattergram. Platelets contained in the specimen scarcely have fluorescent intensity, and thus their emergence location is the location corresponding to low values of fluorescent intensity which almost overlaps on the horizontal axis. Thus, they were not displayed as a clear population on the scattergram of Fig. 14. Erythrocytes in the specimen scarcely have fluorescent intensity. In addition, the erythrocytes are larger in size than the agglutinated triple particles. Thus, they are scaled out from the display range on the scattergram in Fig. 14.

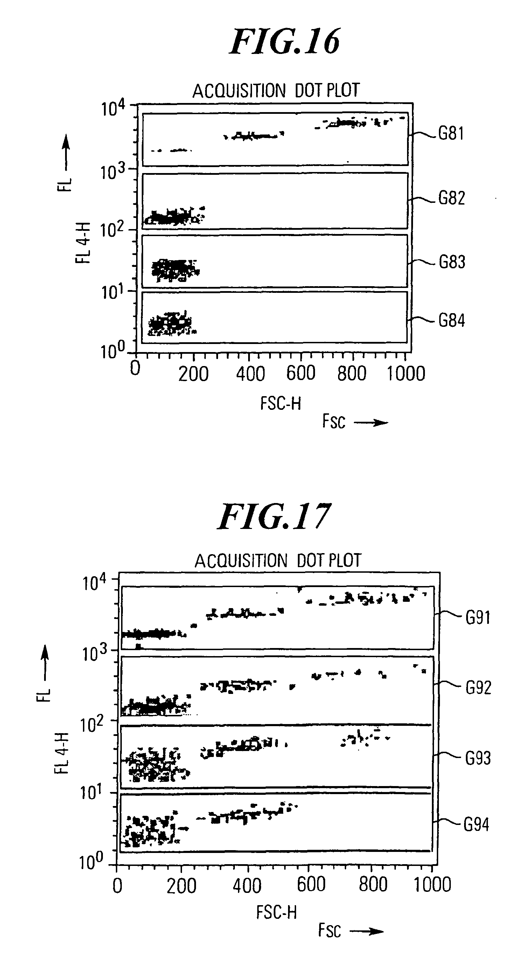

The region G6 was set to surround respective populations of the carrier particles which emerged on the scattergram and particle size distribution in the region G6 was obtained. Fig. 15 is a histogram corresponding to region G6 in Fig. 14. The vertical axis denotes a frequency (particle number) and the horizontal axis denotes the forward scattered light intensity (i.e., sizes of the particles). The numbers of single particles M and agglutinated particles P and the sum of these T were obtained on the basis of this histogram, and the agglutination rates, P/T of the particles which emerged in the region G6 were calculated. Likewise, the agglutination rates were obtained for the respective samples prepared using the specimens at other concentrations of the specimen series containing the HBs antigen and for respective samples prepared using the specimens of other types of the specimen series.

In the following Table 1, agglutination rates (P/T) are shown for the particles in the fluorescent latex reagent for detection of the HBs antigen obtained by measuring the samples prepared using respective specimens of the specimen series containing the HBs antigen (HBs 1 through HBs 7).

**[Table 1]**

| Specimen | Agglutination rate |
|---|---|
| | (P/T%) |
| HBs 1 | 0.37 |
| HBs 2 | 0.78 |
| HBs 3 | 1.80 |
| HBs 4 | 4.91 |
| HBs 5 | 13.46 |
| HBs 6 | 29.68 |
| HBs 7 | 45.31 |

In addition, in the following Table 2, the agglutination rates (P/T) are shown for the particles in the fluorescent latex reagent for detection of the anti-TP antibody obtained by measuring the samples prepared using respective specimens of the specimen series containing the anti-TP antibody (TP 1 through TP 7).

**[Table 2]**

| Specimen | Agglutination rate |
|---|---|
| | (P/T%) |
| TP 1 | 0.41 |
| TP 2 | 1.02 |
| TP 3 | 1.63 |
| TP 4 | 4.52 |
| TP 5 | 12.77 |
| TP 6 | 27.90 |
| TP 7 | 43.85 |

In addition, in the following Table 3, the agglutination rates (P/T) are shown for the particles in the fluorescent latex reagent for detection of the AFP antigen obtained by measuring the samples prepared using respective specimens of the specimen series containing the AFP antigen (AFP 1 through AFP 7).

**[Table 3]**

| Specimen | Agglutination rate |
|---|---|
| | (P/T%) |
| AFP 1 | 0.31 |
| AFP 2 | 1.15 |
| AFP 3 | 2.58 |
| AFP 4 | 6.87 |
| AFP 5 | 17.67 |
| AFP 6 | 36.23 |
| AFP 7 | 52.64 |

In addition, in the following Table 4, the agglutination rates (P/T) are shown of the particles in the fluorescent latex reagent for detection of the CEA antigen obtained by measuring the samples prepared using respective specimens of the specimen series containing the CEA antigen (CEA 1 through 7).

**[Table 4]**

| Specimen | Agglutination rate |
|---|---|
| | (P/T%) |
| CEA 1 | 0.40 |
| CEA 2 | 1.06 |
| CEA 3 | 2.50 |
| CEA 4 | 7.38 |
| CEA 5 | 18.75 |
| CEA 6 | 37.11 |
| CEA 7 | 51.23 |

As shown by the measured results above, the agglutination rates of the particles in the fluorescent latex reagent for each measuring parameter are increased depending on the concentration of the antigen (antibody) contained in the sample. In addition, in each measuring parameter, different agglutination rates were obtained between specimens containing and specimens not containing the antigen (antibody). From these results, it can be said that the antigen (antibody) concentration of each immunological parameter can be obtained by making a standard curve from the agglutination rates for each measuring parameter and converting to concentration on the basis of the standard curve.

### 4. Multiple parameter simultaneous measurement

The measuring results by FACSCalibur using multiple types of fluorescent latex reagents for one specimen are shown. The specimens which were measuring objects were prepared as follows.

### Specimens

As was the case with the above-described single parameter measurement, a total of 28 specimens (i.e., the specimens containing the HBs antigen, HBs 1 through HBs 7, the specimens containing the anti-TP antibody, TP 1 through TP 7, the specimens containing the AFP antigen, AFP 1 through AFP 7, and the specimens containing the CEA antigen, CEA 1 through CEA 7) were used. In addition, the specimen S1 in which four types of antigens or antibody (i.e., the HBs antigen, anti-TP antibody, AFP antigen and CEA antigen) was prepared in human normal whole blood such that the concentration was the same as that in 4 of the specimen series above (i.e., the specimen S1 contains 9 U/mL of HBs antigen, 103.1 mIU of anti-TP antibody, 32 ng/mL of AFP antigen and 8 ng/mL of CEA antigen). The specimen S2 was prepared such that the concentration was the same as that in 7 (i.e., the specimen S2 contains 243 U/mL of HBs antigen, 1610 mIU of anti-TP antibody, 2048 ng/mL of AFP antigen and 512 ng/mL of CEA antigen).

### Measurement

For 40 µl of each specimen, 10 µl each of the fluorescent latex reagent for detection of the HBs antigen, the fluorescent latex reagent for detection of the anti-TP antibody, the fluorescent latex reagent for detection of the AFP antigen and the fluorescent latex reagent for detection of the CEA antigen (total 40 µl of the fluorescent latex reagents) and 320 µl of the reaction buffer were incubated at 45°C for 15 min. The mixture was subsequently diluted with 10 mM PBS buffer to render the sample for measurement. As above, the fluorescence intensity and the forward scattered light intensity were obtained from the sample for measurement, and a scattergram was created using FACSCalibur.

### Analysis

Fig. 16 is a two dimensional scattergram obtained by measuring a sample prepared by adding four types of fluorescent latex reagents (e.g., the fluorescent latex reagent for detection of the HBs antigen, the fluorescent latex reagent for detection of the anti-TP antibody, the fluorescent latex reagent for detection of the AFP antigen, and the fluorescent latex reagent for detection of the CEA antigen) to HBs 4 which was the specimen containing the HBs antigen. The vertical axis denotes fluorescent intensity and the horizontal axis denotes forward scattered light intensity. Due to the difference in the fluorescence intensity, the particles in the four types of fluorescent latex reagents (e.g., the fluorescent latex reagent for detection of the HBs antigen, the fluorescent latex reagent for detection of the anti-TP antibody, the fluorescent latex reagent for detection of the AFP antigen, and the fluorescent latex reagent for detection of the CEA antigen) emerge segregated in the direction of the vertical axis. The region G81 is set to contain the populations of single particles, agglutinated double particles and agglutinated triple particles in the fluorescent latex reagent for detection of the HBs antigen and the particle size distribution of the particles in the region G81 is obtained. Likewise, for fluorescent latex reagents such as the fluorescent latex reagent for the detection of the anti-TP antibody, the fluorescent latex reagent for the detection of the AFP antigen, and the fluorescent latex reagent for the detection of the CEA antigen, the regions G82, G83 and G84 were set, respectively, and a histogram of the particles in each region was created to obtain the particle size distribution. From this histogram, the numbers of single particles M and the agglutinated particles P and the sum of these T were obtained and the agglutination rate P/T of the particles which emerged in each region was calculated.

Fig. 17 is a two dimensional scattergram obtained by measuring the sample prepared by adding four types of fluorescent latex reagents to the specimen S1. The vertical axis denotes fluorescent intensity and the horizontal axis denotes forward scattered light intensity. Due to the difference in the fluorescence intensity, the particles in the four types of fluorescent latex reagents (e.g., the fluorescent latex reagent for the detection of the HBs antigen, the fluorescent latex reagent for the detection of the anti-TP antibody, the fluorescent latex reagent for the detection of the AFP antigen, and the fluorescent latex reagent for the detection of the CEA antigen) emerge segregated in the direction of the vertical axis. In addition, in each fluorescent latex reagent, due to the difference in the forward scattered light intensity, non-agglutinated single particles, agglutinated double particles and agglutinated triple particles are segregated to make populations, which emerge on the scattergram. The region G91 is set to contain the populations of single particles, agglutinated double particles and agglutinated triple particles in the fluorescent latex reagent for detection of the HBs antigen and the particle size distribution of the particles in the region G91 is obtained. Likewise, for respective fluorescent latex reagents (e.g., the fluorescent latex reagent for the detection of the anti-TP antibody, the fluorescent latex reagent for the detection of the AFP antigen and the fluorescent latex reagent for the detection of the CEA antigen), the regions G92, G93 and G94 were set, respectively, and a histogram of the particles in each region was created.

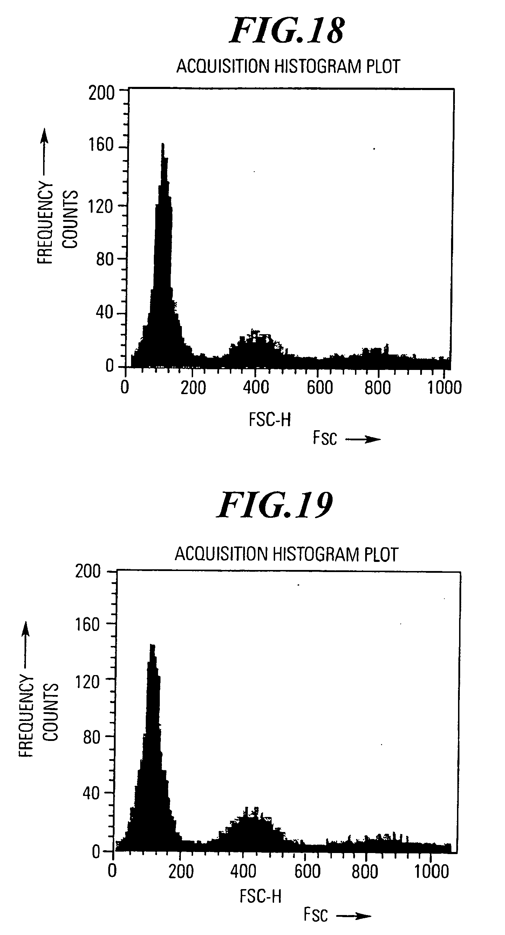

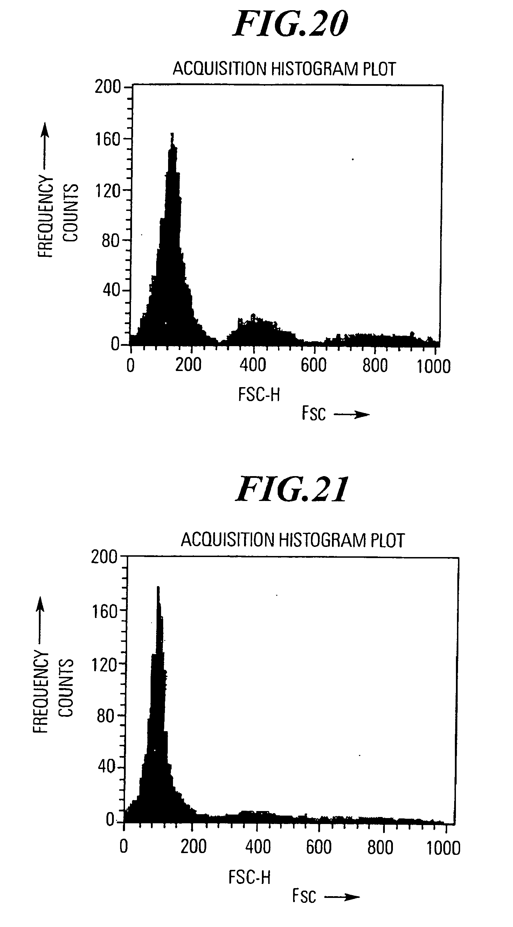

Figs. 18, 19, 20 and 21 are histograms corresponding to the regions G91, 92, 93 and 94, respectively. The vertical axes denotes frequency (number of particles) and the horizontal axis denotes forward scattered light intensity (i.e., size of particles). The particle size distribution in each region was obtained based on these histograms. From them, the numbers of single particles M and the agglutinated particles P and the sum thereof T were obtained. The agglutination rate P/T of the particles which emerged in each region was calculated.

The agglutination rates (P/T) of the particles in the fluorescent latex reagents in respective measuring parameters obtained by measuring the samples prepared from the specimens HBs 1 through HBs 7 are shown in the following Table 5.

**[Table 5]**

| Specimen | Particle agglutination rate (P/T%) in the fluorescent latex reagent of each measuring parameter | | | |
|---|---|---|---|---|
| | HBs antigen | Anti-TP | AFP antigen | CEA antigen |
| | | antibody | | |
| HBs 1 | 0.37 | 0.41 | 0.32 | 0.41 |
| HBs 2 | 0.84 | 0.41 | 0.32 | 0.41 |
| HBs 3 | 2.01 | 0.41 | 0.32 | 0.41 |
| HBs 4 | 5.14 | 0.41 | 0.32 | 0.41 |
| HBs 5 | 13.37 | 0.41 | 0.32 | 0.41 |
| HBs 6 | 29.37 | 0.41 | 0.32 | 0.41 |
| HBs 7 | 46.57 | 0.41 | 0.32 | 0.41 |

From the measured results above, it is found that the agglutination rates of the particles in the fluorescent latex reagent for detection of the HBs antigen are increased depending on the concentrations of the HBs antigen contained in the specimens, whereas the agglutination rates of the particles in other fluorescent latex reagents are not changed regardless of the concentrations of the HBs antigen contained in the specimens.

The agglutination rates (P/T) of the particles in the fluorescent latex reagents in respective measuring parameters obtained by measuring the specimens TP 1 through TP 7 are shown in the following Table 6.

**[Table 6]**

| Specimen | Particle agglutination rate(P/T%) in the fluorescent latex reagent of each measuring parameter | | | |
|---|---|---|---|---|
| | HBS antigen | Anti-TP | AFP antigen | CEA antigen |
| | | antibody | | |
| TP 1 | 0.37 | 0.41 | 0.32 | 0.41 |
| TP 2 | 0.37 | 0.98 | 0.32 | 0.41 |
| TP 3 | 0.37 | 1.52 | 0.32 | 0.41 |
| TP 4 | 0.37 | 4.65 | 0.32 | 0.41 |
| TP 5 | 0.37 | 12.94 | 0.32 | 0.41 |
| TP 6 | 0.37 | 27.82 | 0.32 | 0.41 |
| TP 7 | 0.37 | 43.30 | 0.32 | 0.41 |

From the measured results above, it is found that the agglutination rates of the particles in the fluorescent latex reagent for detection of the anti-TP antibody are increased depending on the concentrations of the anti-TP antibody contained in the specimens, whereas the agglutination rates of the particles in other fluorescent latex reagents are not changed regardless of the concentrations of the anti-TP antibody contained in the specimens.

The agglutination rates (P/T) of the particles in the fluorescent latex reagents in respective measuring parameters obtained by measuring the specimens AFP 1 through AFP 7 are shown in the following Table 7.

**[Table 7]**

| Specimen | Particle agglutination rate (P/T%) in the fluorescent latex reagent of each measuring parameter | | | |
|---|---|---|---|---|
| | HBs antigen | Anti-TP | AFP antigen | CEA antigen |
| | | antibody | | |
| AFP 1 | 0.37 | 0.41 | 0.32 | 0.41 |
| AFP 2 | 0.37 | 0.41 | 1.12 | 0.41 |
| AFP 3 | 0.37 | 0.41 | 2.52 | 0.41 |
| AFP 4 | 0.37 | 0.41 | 7.06 | 0.41 |
| AFP 5 | 0.37 | 0.41 | 17.61 | 0.41 |
| AFP 6 | 0.37 | 0.41 | 36.36 | 0.41 |
| AFP 7 | 0.37 | 0.41 | 51.31 | 0.41 |

From the measured results above, it is found that the agglutination rates of the particles in the fluorescent latex reagent for detection of the AFP antigen are increased depending on the concentrations of the AFP antigen contained in the specimens, whereas the agglutination rates of the particles in other fluorescent latex reagents are not changed regardless of the concentrations of the AFP antigen contained in the specimens.

The agglutination rates (P/T) of the particles in the fluorescent latex reagents in respective measuring parameters obtained by measuring the specimens CEA 1 through CEA 7 are shown in the following Table 8.

**[Table 8]**

| Specimen | Particle agglutination rate (P/T %) in the fluorescent latex reagent of each measuring parameter | | | |
|---|---|---|---|---|
| | HBs antigen | Anti-TP | AFP antigen | CEA antigen |
| | | antibody | | |
| CEA 1 | 0.37 | 0.41 | 0.32 | 0.41 |
| CEA 2 | 0.37 | 0.41 | 0.32 | 1.13 |
| CEA 3 | 0.37 | 0.41 | 0.32 | 2.50 |
| CEA 4 | 0.37 | 0.41 | 0.32 | 7.13 |
| CEA 5 | 0.37 | 0.41 | 0.32 | 18.71 |
| CEA 6 | 0.37 | 0.41 | 0.32 | 36.65 |
| CEA 7 | 0.37 | 0.41 | 0.32 | 51.42 |

From the measured results above, it is found that the agglutination rates of the particles in the fluorescent latex reagent for detection of the CEA antigen are increased depending on the concentrations of the CEA antigen contained in the specimens, whereas the agglutination rates of the particles in other fluorescent latex reagents are not changed regardless of the concentrations of the CEA antigen contained in the specimens.

The agglutination rates (P/T) of the particles in the fluorescent latex reagents in respective measuring parameters obtained by measuring S1 and S2 are shown in the following Table 9.

**[Table 9]**

| Specimen | Particle agglutination rate (P/T%) in the fluorescent latex reagent of each measuring parameter | | | |
|---|---|---|---|---|
| | HBs antigen | Anti-TP antibody | AFP antigen | CEA antigen |
| | | | | |
| S1 | 5.12 | 4.64 | 7.02 | 7.10 |
| S2 | 46.28 | 43.19 | 51.08 | 51.54 |

As indicated in the measured results above, the agglutination rate in each parameter was obtained using whole blood as the specimen by simultaneously reacting four parameters in one reaction system.

### 5. Simultaneous measurement of infection marker-four parameters

Using the automatic immunoassay apparatus 100, immunoassays of four parameters (anti-TP antibody, HBs antigen, anti-HCV antibody, and anti-HIV antibody) which are infection markers were carried out. The specimens were prepared as follows.

### Specimens

Using calibrators (a specimen dilution liquid and specimen dilution liquids where the anti-TP antibody is added at six scales by concentration) for making a standard curve contained in a Ranreamreagent kit forTPmeasurement (Sysmex Corporation), a specimen series was prepared containing anti-TP antibody (TP 1 through TP 7). The concentration of the anti-TP antibody in each specimen in the specimen series containing the anti-TP antibody is as follows:
TP 1: 0 mIU, TP 2: 16.5 mIU, TP 3: 41.2 mIU, TP 4: 103.1 mIU, TP 5: 257.6 mIU, TP 6: 644 mIU, TP 7: 1610 mIU.

Using calibrators (a specimen dilution liquid and specimen dilution liquids where the HBs antigen is added at six scales concentration) for making a standard curve contained in a Ranream reagent kit for HBsAg measurement (Sysmex Corporation), a specimen series was prepared containing the HBs antigen (HBs 1 through HBs 7). The concentration of the HBs antigen in each specimen in the specimen series containing the HBs antigen is as follows:
HBs 1: 0 U/mL, HBs 2: 1 U/mL, HBs 3: 3 U/mL, HBs 4: 9 U/mL, HBs 5: 27 U/mL, HBs 6: 81 U/mL, HBs 7: 243 U/mL.

Using calibrators (made up of a negative control, cutoff control, and positive control, a total of three specimens) for making the standard curve contained in a Ranream reagent kit for HCV IIex measurement (Sysmex Corporation), a specimen series was prepared containing anti-HCV antibody (HCV 1 through HCV 3). In this specimen series containing anti-HCV antibody (HCV 1 through HCV 3), HCV 3 contains the highest concentration of anti-HCV antibody. In HCV 1, the concentration of anti-HCV antibody is 0 U/mL.

As the specimen series containing anti-HIV antibody at 3 scale concentrations, HIV 1 through HIV 3 were prepared as follows. First, PBSbuffer (pH7.2) including 5.0% (w/v) bovine serum albumin was prepared. This was rendered as HIV 1. Next, rabbit anti-HIV antiserum was diluted with PBS buffer (= HIV 1) prepared above, and this was rendered as HIV 3. In addition, one where HIV 3 was diluted with HIV 1 at 100 fold was rendered as HIV 2. In this specimen series containing anti-HIV antibody (HIV 1 to HIV 3), HIV 3 contains the highest concentration of anti-HIV antibody. In HIV 1, the concentration of anti-HCV antibody is 0 U/mL.

### Measurement

The reagent vessel 227 in which the fluorescent latex reagents for simultaneous measurement of infection marker-four parameters were placed and the reagent vessel 231 in which the reaction buffer is placed were set at the reagent setting portion 222 located at the sample preparing portion 200 of the automatic immunoassay apparatus 100. The specimen vessel 225 in which the specimen 225 was placed was set at the specimen setting portion 221 and the automatic immunoassay apparatus 100 was operated as described above to measure each specimen.

### Analysis results

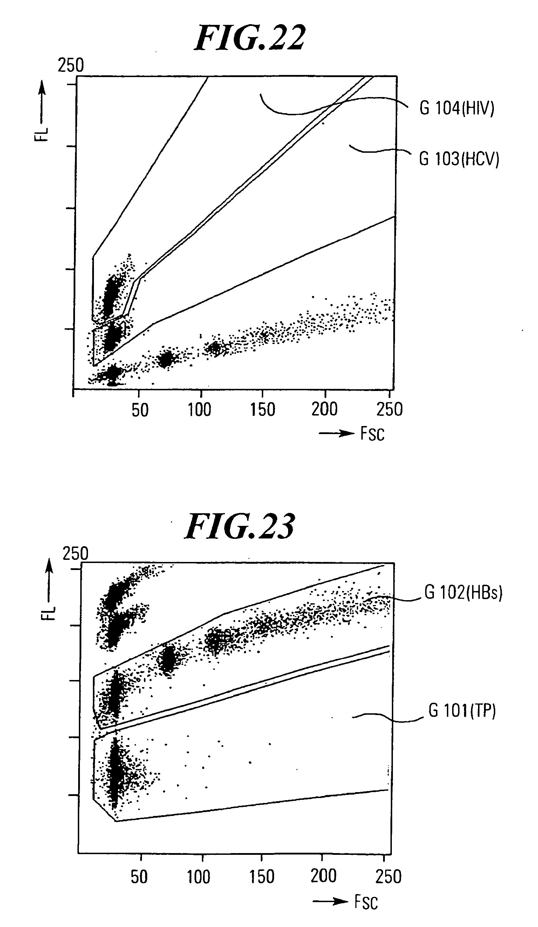

Figs. 22 and 23 are two dimensional scattergrams obtained by measuring HBs 7 which is the specimen containing the HBs antigen. The horizontal axis denotes Fsc and the vertical axis denotes Fl. The scattergrams in Figs. 22 and 23 are those where the results measured from the same specimen were output by changing the display method of the vertical axis. Due to the difference in fluorescent intensity, four types of fluorescent latex particles (i.e., the fluorescent latex particles for detection of the anti-TP antibody, the fluorescent latex particles for detection of the HBs antigen, the fluorescent latex particles for detection of the anti-HCV antibody, and the fluorescent latex particles for detection of the anti-HIV antibody) are segregated in the direction of the vertical axis and emerge in the predetermined regions, respectively. The fluorescent latex particles for detection of the anti-TP antibody, the fluorescent latex particles for detection of the HBs antigen, the fluorescent latex particles for detection of the anti-HCV antibody and the fluorescent latex particles for detection of the anti-HIV antibody emerge in the regions G101, G102, G103 and G104, respectively.

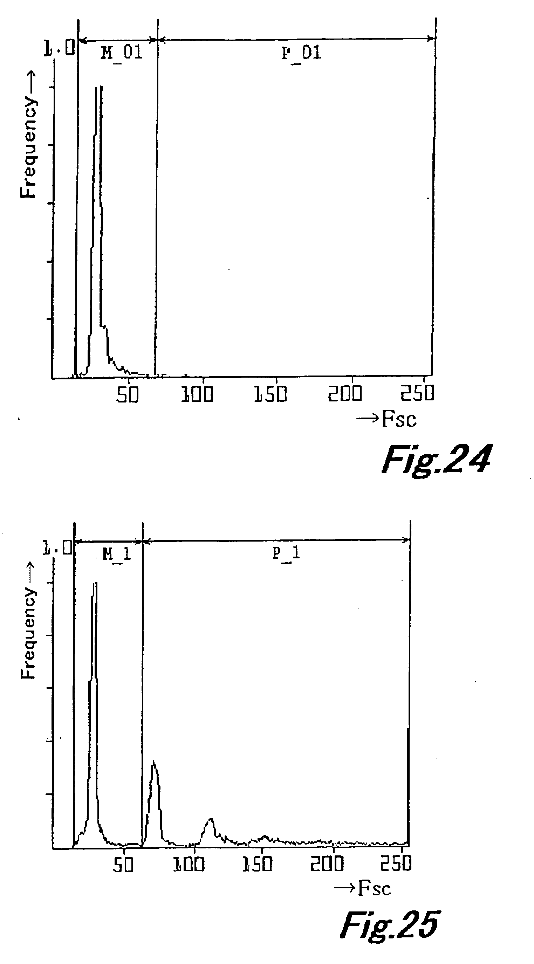

Figs. 24, 25, 26, and 27 are histograms corresponding to the regions G101, G102, G103 and G104 in the two dimensional scattergrams in Fig. 22 and Fig. 23, respectively. The vertical axis denotes frequency (i.e., particle numbers) and the horizontal axis denotes forward scattered light intensity (i.e., sizes of particles).

The agglutination rates (P/T) of the fluorescent latex particles in respective measuring parameters obtained by measuring the samples prepared from the specimens TP 1 through TP 7 are shown in the following Table 10.

**[Table 10]**

| Specimen | Particle agglutination rate (P/T%) in the fluorescent latex particles of each measuring parameter | | | |
|---|---|---|---|---|
| | Anti-TP | HBs antigen | Anti-HCV | Anti-HIV |
| | antibody | | antibody | antibody |
| TP 1 | 0.95 | 0.37 | 0.32 | 0.41 |
| TP 2 | 4.83 | 0.37 | 0.32 | 0.41 |
| TP 3 | 10.34 | 0.37 | 0.32 | 0.41 |
| TP 4 | 20.80 | 0.37 | 0.32 | 0.41 |
| TP 5 | 36.03 | 0.37 | 0.32 | 0.41 |
| TP 6 | 50.06 | 0.37 | 0.32 | 0.41 |
| TP 7 | 60.04 | 0.37 | 0.32 | 0.41 |

From the measured results above, it is found that the agglutination rates of the fluorescent latex particles for detection of the anti-TP antibody are increased depending on the concentrations of anti-TP antibody contained in the specimens, whereas the agglutination rates of other fluorescent latex particles are not changed regardless of the concentrations of anti-TP antibody contained in the specimens.

The agglutination rates (P/T) of the particles in the fluorescent latex particles in respective measuring parameters obtained by measuring the samples prepared from the specimens HBs 1 through HBs 7 are shown in the following Table 11.

**[Table 11]**

| Specimen | Particle agglutination rate (P/T %) in the fluorescent latex particles of each measuring parameter | | | |
|---|---|---|---|---|
| | Anti-TP | HBs antigen | Anti-HCV | Anti-HIV |
| | antibody | | antibody | antibody |
| HBs 1 | 0.41 | 0.70 | 0.32 | 0.41 |
| HBs 2 | 0.41 | 2.22 | 0.32 | 0.41 |
| HBs 3 | 0.41 | 4.93 | 0.32 | 0.41 |
| HBs 4 | 0.41 | 12.51 | 0.32 | 0.41 |
| HBs 5 | 0.41 | 24.57 | 0.32 | 0.41 |
| HBs 6 | 0.41 | 38.35 | 0.32 | 0.41 |
| HBs 7 | 0.41 | 48.09 | 0.32 | 0.41 |

From the measured results above, it is found that the agglutination rates of the fluorescent latex particles for detection of the HBs antigen are increased depending on the concentrations of the HBs antigen contained in the specimens, whereas the agglutination rates of other fluorescent latex particles are not changed regardless of the concentrations of the HBs antigen contained in the specimens.

The agglutination rates (P/T) of the particles in the fluorescent latex particles in respective measuring parameters obtained by measuring the specimens HCV 1 through HCV 3 are shown in the following Table 12.

**[Table 12]**

| Specimen | Particle agglutination rate (P/T%) in the fluorescent latex particles of each measuring parameter | | | |
|---|---|---|---|---|
| | Anti-TP | HBs antigen | Anti-HCV | Anti-HIV |
| | antibody | | antibody | antibody |
| HCV 1 | 0.41 | 0.37 | 0.82 | 0.41 |
| HCV 2 | 0.41 | 0.37 | 1.46 | 0.41 |
| HCV 3 | 0.41 | 0.37 | 26.38 | 0.41 |

From the measured results above, it is found that the agglutination rates of the fluorescent latex particles for detection of the anti-HCV antibody are increased depending on the concentrations of anti-HCV antibody contained in the specimens, whereas the agglutination rates of other fluorescent latex particles are not changed regardless of the concentrations of anti-HCV antibody contained in the specimens.

The agglutination rates (P/T) of the particles in the fluorescent latex particles in respective measuring parameters obtained by measuring the specimens HIV 1 through HIV 3 are shown in the following Table 13.

**[Table 13]**

| Specimen | Particle agglutination rate (P/T%) in the fluorescent latex particles of each measuring parameter | | | |
|---|---|---|---|---|
| | Anti-TP | HBs antigen | Anti-HCV | Anti-HIV |
| | antibody | | antibody | antibody |
| HIV 1 | 0.41 | 0.37 | 0.32 | 1.74 |
| HIV 2 | 0.41 | 0.37 | 0.32 | 3.62 |
| HIV 3 | 0.41 | 0.37 | 0.32 | 43.53 |

From the measured results above, it is found that the agglutination rates of the fluorescent latex particles for detection of the anti-HIV antibody are increased depending on the concentrations of anti-HIV antibody contained in the specimens, whereas the agglutination rates of other fluorescent latex particles are not changed regardless of the concentrations of anti-HIV antibody contained in the specimens.

### 6. Simultaneous measurement of tumor marker-four parameters

Using the automatic immunoassay apparatus 100, immunoassays of four parameters which were tumor markers (FRN antigen, CEA antigen, AFP antigen, and PSA antigen) were carried out. The specimens were prepared as follows.

### Specimens

Using calibrators (a specimen dilution liquid and specimen dilution liquids where FRN antigen is added at six scales by concentration) for making a standard curve contained in a Ranream reagent kit for FRN measurement (Sysmex Corporation), a specimen series was prepared containing FRN antigen (FRN 1 through FRN 7). The concentration of FRN antigen in each specimen in the specimen series containing FRN antigen is as follows:
FRN 1:0ng/mL, FRN2:1.25ng/mL, FRN 3: 5ng/mL, FRN 4:20ng/mL, FRN 5:80ng/mL, FRN 6:320ng/mL, FRN 7:1280ng/mL.

Using calibrators (a specimen dilution liquid and specimen dilution liquids where CEA antigen is added at six scales by concentration) formakinga standard curve contained in a Ranream reagent kit for CEAmeasurement (Sysmex Corporation), a specimen series was prepared containing CEA antigen (CEA 1 through CEA 7). The concentration of CEA antigen in each specimen in the specimen series containing the CEA antigen is as follows:
CEA 1:0ng/mL, CEA 2:0.5ng/mL, CEA 3: 2ng/mL, CEA 4 : 8ng/mL, CEA 5:32ng/mL, CEA 6:128ng/mL, CEA 7:512ng/mL.

Using calibrators (a specimen dilution liquid and specimen dilution liquids where the AFP antigen is added at six scales by concentration) for making a standard curve contained in a Ranream reagent kit for AFP measurement (Sysmex Corporation), a specimen series was prepared containing the AFP antigen (AFP 1 through AFP 7). The concentration of the AFP antigen in each specimen in the specimen series containing the AFP antigen is as follows:
AFP 1:0ng/mL, AFP 2:2ng/mL, AFP 3:8ng/mL, AFP 4:32ng/mL, AFP 5:128ng/mL, AFP 6:512ng/mL, AFP 7:2048ng/mL.

Using calibrators (a specimen dilution liquid and specimen dilution liquids where PSA antigen is added at six scales by concentration) for making a standard curve contained in a Ranream reagent kit for PSAmeasurement (Sysmex Corporation), a specimen series was prepared containing the PSA antigen (PSA 1 through PSA 7). The concentration of the PSA antigen in each specimen in the specimen series containing the PSA antigen is as follows:
PSA 1:0ng/mL, PSA 2:0.125ng/mL, PSA 3:0.5ng/mL, PSA 4:2ng/mL, PSA 5:8ng/mL, PSA 6:32ng/mL, PSA 7:128ng/mL.

### Measurement

The reagent vessel 227 in which the fluorescent latex reagent for simultaneous measurement of tumor marker-four parameters prepared above was placed and the reagent vessel 231 in which the reaction buffer was placed were set at the reagent setting portion 222 located at the sample preparing portion 200 of the automatic immunoassay apparatus 100. The specimen vessel 225 in which the specimen was placed was set at the specimen setting portion 221 and the automatic immunoassay apparatus 100 was operated as described above to measure each specimen.

### Analysis results

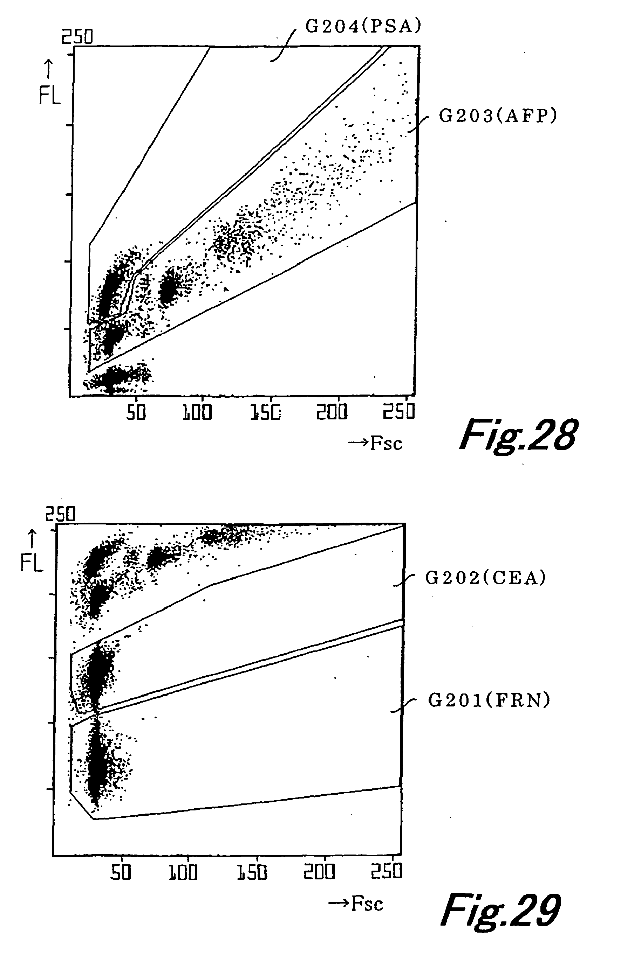

Figs. 28 and 29 are two dimensional scattergrams obtained by measuring AFP 7 which is the specimen containing AFP. The horizontal axis denotes Fsc and the vertical axis denotes FL. The two dimensional scattergrams in Figs. 28 and 29 correspond to those in which the results measured from the same specimen were output by changing the display method of the vertical axis. Due to the difference in fluorescent intensity, four types of fluorescent latex particles (i.e., the fluorescent latex particles for detection of the FRN antigen, the fluorescent latex particles for detection of the CEA antigen, the fluorescent latex particles for detection of the AFP antigen and the fluorescent latex particles for detection of the PSA antigen) are segregated in the direction of the vertical axis and emerge in the predetermined regions, respectively. The fluorescent latex particles for detection of the FRN antigen, the fluorescent latex particles for detection of the CEA antigen, the fluorescent latex particles for detection of the AFP antigen and the fluorescent latex particles for detection of the PSA antigen emerge in the regions G201, G202, G203 and G204, respectively.

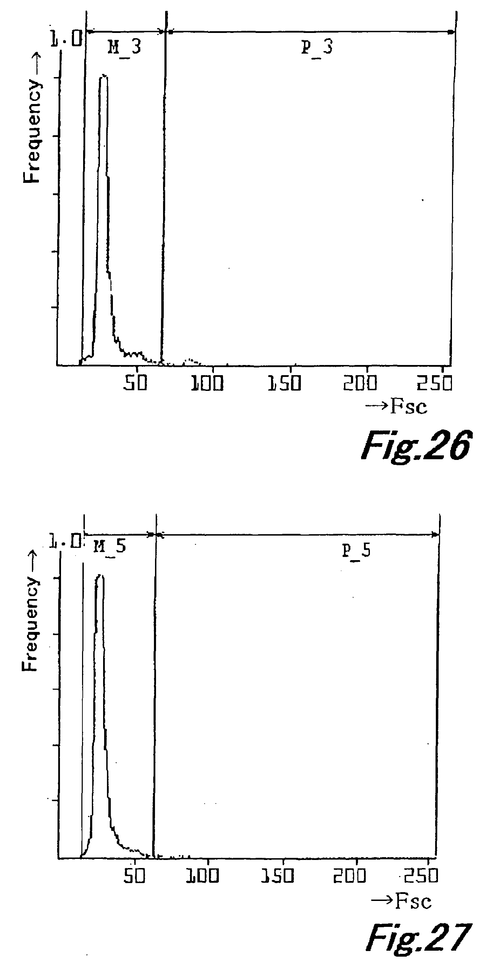

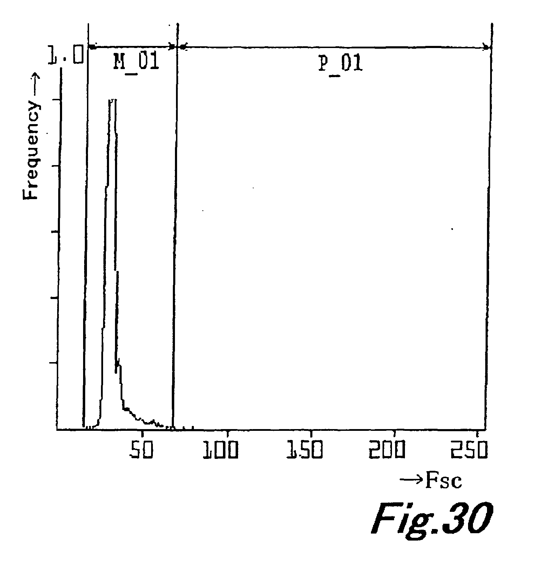

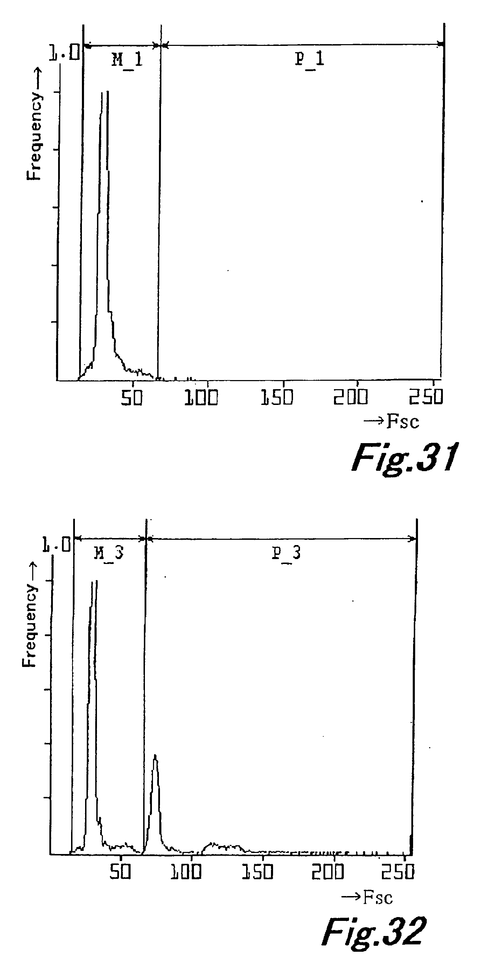

Figs. 30, 31, 32, and 33 are the histograms corresponding to the regions G201, G202, G203 and G204 in the two dimensional scattergrams in Figs. 26 and 27, respectively. The vertical axis denotes frequency (i.e., particle numbers) and the horizontal axis denotes forward scattered light intensity (i.e., sizes of particles).

The agglutination rates (P/T) of the fluorescent latex particles in respective measuring parameters obtained by measuring the samples prepared from the specimens FRN 1 through FRN 7 are shown in the following Table 14.

**[Table 14]**

| Specimen | Particle agglutination rate (P/T%) in the fluorescent latex particles of each measuring parameter | | | |
|---|---|---|---|---|
| | FRN antigen | CEA antigen | AFP antigen | PSA antigen |
| FRN 1 | 0.28 | 0.37 | 0.32 | 0.75 |
| FRN 2 | 0.57 | 0.37 | 0.32 | 0.75 |
| FRN 3 | 1.59 | 0.37 | 0.32 | 0.75 |
| FRN 4 | 5.35 | 0.37 | 0.32 | 0.75 |
| FRN 5 | 17.3 | 0.37 | 0.32 | 0.75 |
| FRN 6 | 46.02 | 0.37 | 0.32 | 0.75 |
| FRN 7 | 62.18 | 0.37 | 0.32 | 0.75 |

From the measured results above, it is found that the agglutination rates of the fluorescent latex particles for detection of the FRN antigen are increased depending on the concentrations of the FRN antigen contained in the specimens, whereas the agglutination rates of other fluorescent latex particles are not changed regardless of the concentrations of the FRN antigen contained in the specimens.

The agglutination rates (P/T) of the fluorescent latex particles in respective measuring parameters obtained by measuring the samples prepared from the specimens CEA 1 through CEA 7 are shown in the following Table 15.

**[Table 15]**

| Specimen | Particle agglutination rate (P/T%) in the fluorescent latex particles of each measuring parameter | | | |
|---|---|---|---|---|
| | FRN antigen | CEA antigen | AFP antigen | PSA antigen |
| CEA 1 | 0.28 | 0.70 | 0.32 | 0.75 |
| CEA 2 | 0.28 | 1.06 | 0.32 | 0.75 |
| CEA 3 | 0.28 | 2.70 | 0.32 | 0.75 |
| CEA 4 | 0.28 | 7.59 | 0.32 | 0.75 |
| CEA 5 | 0.28 | 18.31 | 0.32 | 0.75 |
| CEA 6 | 0.28 | 37.81 | 0.32 | 0.75 |
| CEA 7 | 0.28 | 51.22 | 0.32 | 0.75 |

From the measured results above, it is found that the agglutination rates of the fluorescent latex particles for detection of the CEA antigen are increased depending on the concentrations of the CEA antigen contained in the specimens, whereas the agglutination rates of other fluorescent latex particles are not changed regardless of the concentrations of the CEA antigen contained in the specimens.

The agglutination rates (P/T) of the fluorescent latex particles in respective measuring parameters obtained by measuring the specimens AFP 1 through AFP 7 are shown in the following Table 16.

**[Table 16]**

| Specimen | Particle agglutination rate (P/T %) in the fluorescent latex particles of each measuring parameter | | | |
|---|---|---|---|---|
| | FRN antigen | CEA antigen | AFP antigen | PSA antigen |
| AFP 1 | 0.28 | 0.70 | 0.32 | 0.75 |
| AFP 2 | 0.28 | 0.70 | 1.15 | 0.75 |
| AFP 3 | 0.28 | 0.70 | 2.58 | 0.75 |
| AFP 4 | 0.28 | 0.70 | 6.87 | 0.75 |
| AFP 5 | 0.28 | 0.70 | 17.67 | 0.75 |
| AFP 6 | 0.28 | 0.70 | 36.23 | 0.75 |
| AFP 7 | 0.28 | 0.70 | 52.64 | 0.75 |

From the measured results above, it is found that the agglutination rates of the fluorescent latex particles for detection of the AFP antigen are increased depending on the concentrations of the AFP antigen contained in the specimens, whereas the agglutination rates of other fluorescent latex particles are not changed regardless of the concentrations of the AFP antigen contained in the specimens.

The agglutination rates (P/T) of the fluorescent latex particles in respective measuring parameters obtained by measuring the specimens PSA 1 through PSA 7 are shown in the following Table 17.

**[Table 17]**

| Specimen | Particle agglutination rate (P/T%) in the fluorescent latex particles of each measuring parameter | | | |
|---|---|---|---|---|
| | FRN antigen | CEA antigen | AFP antigen | PSA antigen |
| PSA 1 | 0.28 | 0.70 | 0.32 | 0.75 |
| PSA 2 | 0.28 | 0.70 | 0.32 | 1.02 |
| PSA 3 | 0.28 | 0.70 | 0.32 | 1.78 |
| PSA 4 | 0.28 | 0.70 | 0.32 | 6.06 |
| PSA 5 | 0.28 | 0.70 | 0.32 | 14.13 |
| PSA 6 | 0.28 | 0.70 | 0.32 | 29.66 |
| PSA 7 | 0.28 | 0.70 | 0.32 | 42.38 |

From the measured results above, it is found that the agglutination rates of the fluorescent latex particles for detection of the PSA antigen are increased depending on the concentrations of the PSA antigen contained in the specimens, whereas the agglutination rates of other fluorescent latex reagents are not changed regardless of the concentrations of the PSA antigen contained in the specimens.

In the simultaneous measurement of the infection marker-four parameters described above, the immunoassays of multiple parameters associated with the infections (anti-TP antibody, HBs antigen, anti-HCV antibody and anti-HIV antibody) were simultaneously carried out for one specimen using the fluorescent latex reagent containing the fluorescent latex particles immobilized with TP antigen, the fluorescent latex particles immobilized with anti-HBs antibody, the fluorescent latex particles immobilized with HCV antigen and the fluorescent latex particles immobilized with HIV antigen as the carrier particles. Thus, the combination of multiple types of fluorescent latex particles responding to the parameters associated with infections can provide the reagent for simultaneous measurement of multiple parameters of infection markers.

In addition, in the simultaneous measurement of tumor marker four parameters described above, the immunoassays of multiple parameters associated with the tumors (FRN antigen, CEA antigen, AFP antigen and PSA antigen) were simultaneously carried out for one specimen using the fluorescent latex reagent containing the fluorescent latex particles immobilized with anti-FRN antibody, the fluorescent latex particles immobilized with anti-CEA antibody, the fluorescent latex particles immobilized with anti-AFP antibody and the fluorescent latex particles immobilized with anti-PSA antibody as the carrier particles. Thus, the combination of multiple types of fluorescent latex particles responding to the parameters associated with tumors can provide the reagent for simultaneous measurement of multiple parameters associated with tumor markers.

The above-described reagents for the four parameter simultaneous measurements are useful for measuring multiple parameters associated with infections and tumors in a short time period. However, in the case of simultaneously measuring multiple parameters, the combination of the measuring parameters does not need to be limited to the above. For example, multiple parameters selected of so-called immunological serum test parameters (CRP, RF, ASO, IgG, IgA, IgM, IgE, etc.) may be combined, or multiple parameters selected from endocrine test parameters (TSH, T4, T3, FT4, etc.) may be combined. In addition, multiple parameters selected from blood drug test parameters (PHT, PB, PRM, CBZ, VPA, etc.) maybe combined. The parameters desired to be simultaneously measured may be, of course, appropriately combined regardless of their test categories.

In the above-described simultaneous measurements of the infection marker-four parameters and the tumor marker-four parameters, the suspension in which the multiple types of fluorescent latex particles responding to the multiple parameters were suspended in one liquid beforehand was used as the fluorescent latex reagent. Conventionally, when four parameters are measured, the reagents for the measurement responding to respective parameters are required. However, in the examples in accordance with the present invention, the fluorescent latex reagent for the measurement of respective parameters is made up of one liquid. In addition, only one type of reaction buffer is for the simultaneous measurement of four parameters. Since it is not necessary to prepare the fluorescent latex reagents and the reaction buffers for respective multiple measuring parameters, a space for setting the reagents can be made more compact in the automatic immunoassay apparatus, which consequently leads to downsizing of the whole apparatus.

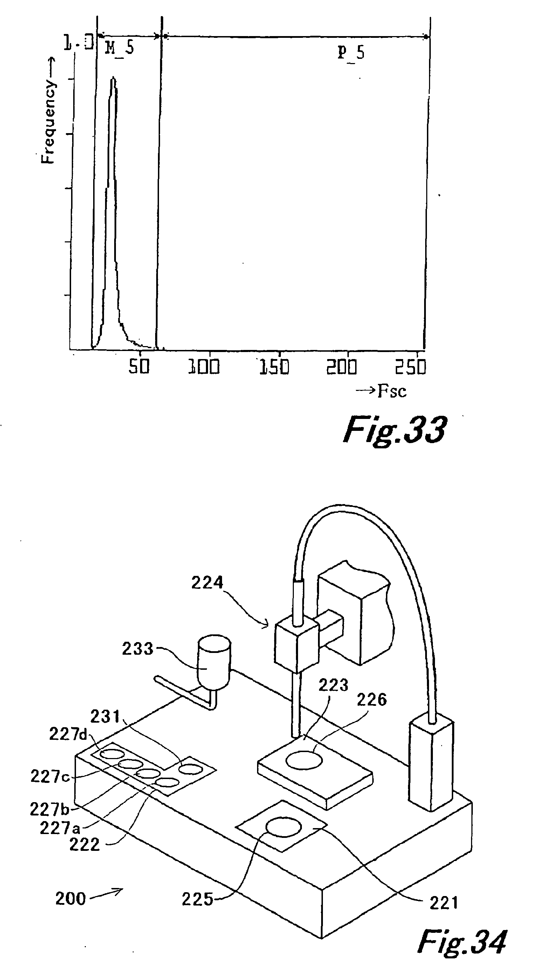

As another configuration of the reagents, the fluorescent latex reagent is prepared for each measuring parameter, the fluorescent latex reagents are configured such that the fluorescent latex reagent depends on the test order, and the desired measuring parameter is selected and combined. Fig. 34 is a drawing showing a modified example of the sample preparing portion of the automatic immunoassay apparatus 100 shown in Fig. 7. The sample preparing portion 200 in Fig. 34 is configured so as to combine multiple types of fluorescent latex reagents and perform the simultaneous measurement of multiple parameters. The reagent setting portion 222 can set the reagent vessels 27a, 27b, 27c and 27d in which the first, second, third and forth fluorescent latex reagents are placed, respectively. The first through fourth fluorescent latex reagents respond to different measuring parameters, respectively, and the fluorescent latex particles contained therein encompass fluorescent dye at different concentrations from one another. The configurations other than the reagent setting portion 222 are the same as those described in Fig. 7.

In this modified example, the automatic immunoassay apparatus 100 can select two operation modes: the four parameter simultaneous measuring mode and the single parameter measuring mode. The operator selects either operation mode using the liquid crystal touch panel 101 prior to the measurement. After selecting the four parameter simultaneous measuring mode, when the starting switch 103 is pushed, the sample preparing portion 200 in Fig. 34 operates as follows in S1 (control of the sample preparing portion) in the entire control of the apparatus.

First, the dispensing device 224 aspirates 80 µl of the reaction buffer from the reagent vessel 231 at the reagent setting portion 222. Next, the dispensing device 224 aspirates 10 µl of the specimen from the specimen vessel 225 set at the specimen setting portion 221, and dispenses the reaction buffer and the specimen aspirated above into the reaction vessel 226 set at the incubator 223. Next, the dispensing device 224 aspirates 2.5 µl of the first fluorescent latex reagent from the reagent vessel 227a at the reagent setting portion 222, and dispenses it into the reaction vessel 226. Next, the dispensing device 224 aspirates 2. 5 µl of the second fluorescent latex reagent from the reagent vessel 227b at the reagent setting portion 222, and dispenses it into the reaction vessel 226. The dispensing device 224 also aspirates each 2.5 µl of the third and fourth fluorescent latex reagents and dispenses them into the reaction vessel 226, similarly. Subsequently, the incubator 223 incubates by shaking/stirring the reaction vessel 226 in which the specimen, the fluorescent latex reagents and the reaction buffer are placed while maintaining a temperature at 45°C for 15 min and reacts the specimen with the reagents to prepare a sample liquid. The dispensing device 224 aspirates the sample liquid after the incubation, and supplies it into the sample vessel 233. A dilution liquid is placed in the sample vessel 233 beforehand and the sample liquid dispensed is diluted 51-fold. Subsequently, the controls of the light detecting portion and the analysis portion are carried out as described in the above examples.

Prior to measurement, when the single parameter measuring mode is selected, the sample preparing portion 200 is controlled as follows. The dispensing device 224 first aspirates the reaction buffer and the specimen and dispenses them into the reaction vessel 226 as above, and subsequently aspirates 10 µl of the fluorescent latex reagent for the ordered measuring parameter and dispenses it into the reaction vessel 226. Other operations are controlled as above.

In accordance with the present invention, even when using the specimen and the whole blood specimen containing particles other than the carrier particles (e.g., erythrocytes, platelets, chylomicrons and bacteria), the antigen (antibody) in the specimen can be detected with excellent accuracy. In accordance with the present invention, even when the carrier particles used are those which overlap in size with particles other than the carrier particles, carrier particles can be differentiated and counted to yield an agglutination level. Thus, the restriction on the sizes of particles is reduced upon selection of the carrier particles. In accordance with the present invention, since no labeling antibody to label the measuring object substance bound to the carrier particles is used, the types of reagents can be reduced.

In addition, the particle agglutination reaction for the multiple measuring object substances can be carried out in one reaction system, and respective measuring object substances can be simultaneously detected with excellent accuracy. Thus, multiple parameters of immunoassays can be carried out in a short time period in the event of an emergency.

In addition, in accordance with the present invention, it becomes possible to downsize the automatic immunoassay apparatus by making the reagents for multiple parameter measurement such as infection markers and tumor markers into one reagent. Both the single parameter measurement and the multiple parameter simultaneous measurement can be performed such that the reagents for respective measuring parameters are used in combination.

The foregoing detailed description and examples have been provided by way of explanation and illustration. Many variations in the presently desirable embodiments illustrated herein will be obvious to one of ordinary skill in the art,

## Claims

1. An immunoassay method comprising:
(a) preparing a measuring sample by mixing a specimen and carrier particles;
wherein the specimen comprises a measuring object substance and coexisting particles;
wherein an antibody or an antigen against the measuring object substance is immobilised on the carrier particles;
wherein the carrier particles have a different physical property than the coexisting particles;
wherein said different physical property is selected from fluorescence, absorbance, phosphorescence, chemiluminescence or biological luminescence, and
wherein the carrier particles are agglutinated when the measuring object substance is present in the specimen;
(b) detecting first and second optical information from the coexisting particles and the carrier particles in the measuring sample, wherein said first optical information is selected from fluorescence, absorbance, phosphorescence, chemiluminescence or biological luminescence, and said second optical information is scattered light;
(c) differentiating the carrier particles from the coexisting particles on the basis of the first optical information;
(d) detecting an agglutination level of the carrier particles on the basis of the second optical information; and
(e) analyzing whether the measuring object substance is present in the specimen or not on the basis of the agglutination level of the carrier particles.

2. An immunoassay method comprising:
(a) preparing a measuring sample by mixing a specimen comprising first and second measuring object substances, and coexisting particles, and first and second carrier particles;
wherein an antibody or an antigen against the first measuring object substance is immobilized on the first carrier particles, and the first carrier particles agglutinate when the first measuring object substance is present;
wherein an antibody or an antigen against the second measuring object substance is immobilized on the second carrier particles, and the second carrier particles agglutinate when the second measuring object substance is present;
wherein the first and second carrier particles comprise different optical information than the coexisting particles; and
wherein the first carrier particles have a different physical property than the second carrier particles, wherein said different physical property is selected from fluorescence, absorbance, phosphorescence, chemiluminescence or biological luminescence;
(b) detecting the first and second optical information from the first and second carrier particles and the coexisting particles in the measuring sample,
wherein said first optical information is selected from fluorescence, absorbance, phosphorescence, chemiluminescence or biological luminescence, and said second optical information is scattered light;
(c) differentiating the first carrier particles, the second carrier particles, and the coexisting particles on the basis of the first optical information;
(d) detecting respective agglutination levels of the first and second carrier particles on the basis of the second optical information; and
(e) analyzing whether at least one of the first and second measuring object substances is present in the specimen or not on the basis of the respective agglutination levels.

3. The method of claim 1 or 2, wherein the specimen is whole blood, and the coexisting particles are erythrocytes or platelets.

4. The method of claim 1 or 2, wherein the first optical information is selected from the group consisting of fluorescence, chemiluminescence, and absorbance.

5. An immunoassay apparatus comprising:
(a) a measuring sample provider for providing a measuring sample wherein the measuring sample is a mixture of a specimen and carrier particles;
wherein the specimen comprises a measuring object substance and coexisting particles;
wherein an antibody or an antigen against the measuring object substance is immobilized on the carrier particles;
wherein the carrier particles have a different physical property than the coexisting particles,
wherein said different physical property is selected from fluorescence, absorbance, phosphorescence, chemiluminescence or biological luminescence; and
wherein the carrier particles are agglutinated when the measuring object substance is present in the specimen;
(b) an optical information detector for detecting first and second optical information from the coexisting particles and the carrier particles in the measuring sample, wherein said first optical information is selected from fluorescence, absorbance, phosphorescence, chemiluminescence or biological luminescence, and said second optical information is scattered light;
(c) a controller which differentiates the carrier particles from the coexisting particles on the basis of the first optical information, calculates an agglutination level of the carrier particles on the basis of the second optical information, and analyzes whether the measuring object substance is present in the specimen or not on the basis of the agglutination level of the carrier particles.

6. An immunoassay apparatus comprising:
(a) a measuring sample provider for providing a measuring sample wherein the measuring sample is a mixture of a specimen and first and second carrier particles,
wherein the specimen comprises first and second measuring object substances and coexisting particles;
wherein an antibody or an antigen against the first measuring object substance is immobilized on the first carrier particles, and the first carrier particles agglutinate when the first measuring object substance is present;
wherein an antibody or an antigen against the second measuring object substance is immobilized on the second carrier particles, and the second carrier particles agglutinate when the second measuring object substance is present; and
wherein the first carrier particles have a different physical property, than the second carrier particles, wherein said different physical property is selected from fluorescence, absorbance, phosphorescence, chemiluminescence or biological luminescence;
(b) an optical information detector for detecting the first and second optical information from the first and second carrier particles, wherein said first optical information is selected from fluorescence, absorbance, phosphorescence, chemiluminescence or biological luminescence, and said second optical information is scattered light;
(c) a controller which differentiates the first carrier particles, the second carrier particles and the coexisting particles on the basis of the first optical information, calculates respective agglutination levels of the first and second carrier particles on the basis of the second optical information; and analyzes whether at least one of the first and second measuring object substances is present in the specimen or not on the basis of the respective agglutination levels.

## Patentansprüche

1. Immunassayverfahren umfassend:
(a) Herstellen einer Messprobe durch Mischen einer Probe mit Trägerpartikeln;
wobei die Probe eine zu messende Zielsubstanz und co-existierende Partikel umfasst;
wobei ein Antikörper oder ein Antigen gegen die zu messende Zielsubstanz auf den Trägerpartikeln immobilisiert ist;
wobei die Trägerpartikel eine andere physikalische Eigenschaft haben als die co-existierenden Partikel;
wobei diese andere physikalische Eigenschaft ausgewählt ist aus Fluoreszenz, Absorption, Phosphoreszenz, Chemilumineszenz oder biologischer Lumineszenz, und
wobei die Trägerpartikel agglutiniert sind, wenn die zu messende Zielsubstanz in der Probe vorhanden ist;
(b) Nachweisen einer ersten und zweiten optischen Information der co-existierenden Partikel und der Trägerpartikel in der Messprobe, wobei die erste optische Information ausgewählt ist aus Fluoreszenz, Absorption, Phosphoreszenz, Chemilumineszenz oder biologischer Lumineszenz, und die zweite optische Information Streulicht ist;
(c) Unterscheiden der Trägerpartikel von den co-existierenden Partikeln auf Grundlage der ersten optischen Information;
(d) Nachweisen eines Agglutinierungsniveaus der Trägerpartikel auf Grundlage der zweiten optischen Information; und
(e) Analysieren auf Grundlage des Agglutinierungsniveaus der Trägerpartikel, ob die zu messende Zielsubstanz in der Probe vorhanden ist oder nicht.

2. Immunassayverfahren umfassend:
(a) Herstellen einer Messprobe durch Mischen einer Probe, die eine erste und zweite zu messende Zielsubstanz und co-existierende Partikel und erste und zweite Trägerpartikel umfasst;
wobei ein Antikörper oder ein Antigen gegen die erste zu messende Zielsubstanz auf den ersten Trägerpartikeln immobilisiert ist und die ersten Trägerpartikel agglutinieren, wenn die erste zu messende Zielsubstanz vorhanden ist;
wobei ein Antikörper oder ein Antigen gegen die zweite zu messende Zielsubstanz auf den zweiten Trägerpartikeln immobilisiert ist und die zweiten Trägerpartikel agglutinieren, wenn die zweite zu messende Zielsubstanz vorhanden ist;
wobei die ersten und zweiten Trägerpartikel verschiedene optische Informationen als die co-existierenden Partikel umfassen; und
wobei die ersten Trägerpartikel eine andere physikalische Eigenschaft haben als die zweiten Trägerpartikel,
wobei die andere physikalische Eigenschaft ausgewählt ist aus Fluoreszenz, Absorption, Phosphoreszenz, Chemilumineszenz oder biologischer Lumineszenz;
(b) Nachweisen der ersten und zweiten optischen Information der ersten und zweiten Trägerpartikel und der co-existierenden Partikel in der Messprobe, wobei diese erste optische Information ausgewählt ist aus Fluoreszenz, Absorption, Phosphoreszenz, Chemilumineszenz oder biologischer Lumineszenz und diese zweite optische Information Streulicht ist;
(c) Unterscheiden der ersten Trägerpartikel, der zweiten Trägerpartikel und der co-existierenden Partikel auf Grundlage der ersten optischen Information;
(d) Nachweisen der jeweiligen Agglutinierungsniveaus der ersten und zweiten Trägerpartikel auf Grundlage der zweiten optischen Information; und
(e) Analysieren auf Grundlage der jeweiligen Agglutinierungsniveaus, ob mindestens eine der ersten und zweiten zu messenden Zielsubstanzen in der Probe vorhanden ist oder nicht.

3. Verfahren gemäß Anspruch 1 oder 2, wobei die Probe Vollblut ist und die co-existierenden Partikel Erythrozyten oder Plättchen sind.

4. Verfahren gemäß Anspruch 1 oder 2, wobei die erste optische Information ausgewählt ist aus der Gruppe bestehend aus Fluoreszenz, Chemilumineszenz und Absorption.

5. Immunassay-Apparat umfassend:
(a) eine Messprobenzufuhr zum Zuführen einer Messprobe, wobei die Messprobe eine Mischung einer Probe und Trägerpartikel ist;
wobei die Probe eine zu messende Zielsubstanz und co-existierende Partikel umfasst;
wobei ein Antikörper oder ein Antigen gegen die zu messende Zielsubstanz auf den Trägerpartikeln immobilisiert ist;
wobei die Trägerpartikel eine andere physikalische Eigenschaft haben als die co-existierenden Partikel,
wobei diese physikalische Eigenschaft ausgewählt ist aus Fluoreszenz, Absorption, Phosphoreszenz, Chemilumineszenz oder biologischer Lumineszenz; und
wobei die Trägerpartikel agglutiniert sind, wenn die zu messende Zielsubstanz in der Probe vorhanden ist;
(b) einen Detektor optischer Informationen zum Nachweisen einer ersten und zweiten optischen Information der co-existierenden Partikel und der Trägerpartikel in der Messprobe, wobei die erste optische Information ausgewählt ist aus Fluoreszenz, Absorption, Phosphoreszenz, Chemilumineszenz oder biologischer Lumineszenz und die zweite optische Information Streulicht ist;
(c) eine Kontrolleinheit, welche die Trägerpartikel von den co-existierenden Partikeln auf Grundlage der ersten optischen Information unterscheidet, das Agglutinierungsniveau der Trägerpartikel auf Grundlage der zweiten optischen Information berechnet und auf Grundlage des Agglutinierungsniveaus der Trägerpartikel analysiert, ob die zu messende Zielsubstanz in der Probe vorhanden ist oder nicht.

6. Immunassay-Apparat umfassend:
(a) eine Messprobenzufuhr zum Zuführen einer Messprobe,
wobei die Messprobe ein Gemisch einer Probe und erster und zweiter Trägerpartikel ist, wobei die Probe eine erste und zweite zu messende Zielsubstanz und co-existierende Partikel umfasst;
wobei ein Antikörper oder ein Antigen gegen die erste zu messende Zielsubstanz auf den ersten Trägerpartikeln immobilisiert ist und die ersten Trägerpartikel agglutinieren, wenn die erste zu messende Zielsubstanz vorhanden ist;
wobei ein Antikörper oder ein Antigen gegen die zweite zu messende Zielsubstanz auf den zweiten Trägerpartikeln immobilisiert ist und die zweiten Trägerpartikel agglutinieren, wenn die zweite zu messende Zielsubstanz vorhanden ist; und
wobei die ersten Trägerpartikel eine andere physikalische Eigenschaft haben als die zweiten Trägerpartikel, wobei die andere physikalische Eigenschaft ausgewählt ist aus Fluoreszenz, Absorption, Phosphoreszenz, Chemilumineszenz oder biologischer Lumineszenz;
(b) einen Detektor optischer Informationen zum Nachweisen der ersten und zweiten optischen Information der ersten und zweiten Trägerpartikel, wobei die erste optische Information ausgewählt ist aus Fluoreszenz, Absorption, Phosphoreszenz, Chemilumineszenz oder biologischer Lumineszenz und die zweite optische Information Streulicht ist;
(c) eine Kontrolleinheit, die die ersten Trägerpartikel, die zweiten Trägerpartikel und die co-existierenden Partikel auf Grundlage der ersten optischen Information unterscheidet, die entsprechenden Agglutinierungsniveaus der ersten und zweiten Trägerpartikel auf Grundlage der zweiten optischen Information berechnet; und auf Grundlage der entsprechenden Agglutinierungsniveaus analysiert, ob mindestens eine der ersten und zweiten zu messenden Zielsubstanzen in der Probe vorhanden ist.

## Revendications

1. Procédé de dosage immunologique comprenant :
(a) préparer un échantillon de mesure en mélangeant un spécimen et des particules porteuses ;
dans lequel le spécimen comprend une substance objet de mesure et des particules coexistantes ;
dans lequel un anticorps ou un antigène contre la substance objet de mesure est immobilisé sur les particules porteuses ;
dans lequel les particules porteuses ont une propriété physique différente des particules coexistantes ;
dans lequel ladite propriété physique différente est choisie parmi la fluorescence, l'absorbance, la phosphorescence, la chimioluminescence ou la luminescence biologique, et
dans lequel les particules porteuses sont agglutinées lorsque la substance objet de mesure est présente dans le spécimen ;
(b) détecter une première et seconde information optique à partir des particules coexistantes et des particules porteuses dans l'échantillon de mesure, dans lequel ladite première information optique est choisie parmi la fluorescence, l'absorbance, la phosphorescence, la chimioluminescence ou la luminescence biologique, et ladite seconde information optique est la lumière diffusée ;
(c) différencier les particules porteuses des particules coexistantes sur la base de la première information optique ;
(d) détecter un niveau d'agglutination des particules porteuses sur la base de la seconde information optique ; et
(e) analyser si la substance objet de mesure est présente dans le spécimen ou non sur la base du niveau d'agglutination des particules porteuses.

2. Procédé de dosage immunologique comprenant :
(a) préparer l'échantillon de mesure en mélangeant un spécimen comprenant une première et seconde substance objet de mesure, et des particules coexistantes et des premières et secondes particules porteuses ;
dans lequel un anticorps ou un antigène contre la première substance objet de mesure est immobilisé sur les premières particules porteuses, et les premières particules porteuses s'agglutinent lorsque la première substance objet de mesure est présente ;
dans lequel un anticorps ou un antigène contre la seconde substance objet de mesure est immobilisé sur les secondes particules porteuses, et les secondes particules porteuses s'agglutinent lorsque la seconde substance objet de mesure est présente ;
dans lequel les premières et secondes particules porteuses comprennent une information optique différente des particules coexistantes ; et
dans lequel les premières particules porteuses ont une propriété physique différente des secondes particules porteuses, dans lequel ladite propriété physique différente est choisie parmi la fluorescence, l'absorbance, la phosphorescence, la chimioluminescence ou la luminescence biologique ;
(b) détecter la première et seconde information optique provenant des premières et secondes particules porteuses et des particules coexistantes dans l'échantillon de mesure, dans lequel ladite première information optique est choisie parmi la fluorescence, l'absorbance, la phosphorescence, la chimioluminescence ou la luminescence biologique, et ladite seconde information optique est la lumière diffusée ;
(c) différencier les premières particules porteuses, les secondes particules porteuses et les particules coexistantes sur la base de la première information optique ;
(d) détecter les niveaux d'agglutination respectifs des premières et secondes particules porteuses sur la base de la seconde information optique ; et
(e) analyser si au moins une de la première et seconde substance objet de mesure est présente dans le spécimen ou non sur la base des niveaux d'agglutination respectifs.

3. Procédé de la revendication 1 ou 2, dans lequel le spécimen est du sang total, et les particules coexistantes sont des érythrocytes ou des plaquettes.

4. Procédé de la revendication 1 ou 2, dans lequel la première information optique est choisie parmi le groupe constitué de la fluorescence, la chimioluminescence, et l'absorbance.

5. Appareil de dosage immunologique comprenant :
(a) un fournisseur d'échantillon de mesure destiné à fournir un échantillon de mesure dans lequel l'échantillon de mesure est un mélange d'un spécimen et de particules porteuses ;
dans lequel le spécimen comprend une substance objet de mesure et des particules coexistantes ;
dans lequel un anticorps ou un antigène contre la substance objet de mesure est immobilisé sur les particules porteuses ;
dans lequel les particules porteuses ont une propriété physique différente des particules coexistantes, dans lequel ladite propriété physique différente est choisie parmi la fluorescence, l'absorbance, la phosphorescence, la chimioluminescence ou la luminescence biologique ; et
dans lequel les particules porteuses sont agglutinées lorsque la substance objet de mesure est présente dans le spécimen ;
(b) un détecteur d'informations optiques destiné à détecter une première et seconde information optique provenant des particules coexistantes et des particules porteuses dans l'échantillon de mesure, dans lequel ladite première information optique est choisie parmi la fluorescence, l'absorbance, la phosphorescence, la chimioluminescence ou la luminescence biologique, et ladite seconde information optique est la lumière diffusée ;
(c) un régulateur qui différencie les particules porteuses des particules coexistantes sur la base de la première information optique, calcule un niveau d'agglutination des particules porteuses sur la base de la seconde information optique, et analyse si la substance objet de mesure est présente dans le spécimen ou non sur la base du niveau d'agglutination des particules porteuses.

6. Appareil de dosage immunologique comprenant :
(a) un fournisseur d'échantillon de mesure destiné à fournir un échantillon de mesure dans lequel l'échantillon de mesure est un mélange d'un spécimen et de premières et secondes particules porteuses,
dans lequel le spécimen comprend une première et seconde substance objet de mesure et des particules coexistantes ;
dans lequel un anticorps ou un antigène contre la première substance objet de mesure est immobilisé sur les premières particules porteuses, et les premières particules porteuses s'agglutinent lorsque la première substance objet de mesure est présente ;
dans lequel un anticorps ou un antigène contre la seconde substance objet de mesure est immobilisé sur les secondes particules porteuses, et les secondes particules porteuses s'agglutinent lorsque la seconde substance objet de mesure est présente ; et
dans lequel les premières particules porteuses ont une propriété physique différente des secondes particules porteuses, dans lequel ladite propriété physique différente est choisie parmi la fluorescence, l'absorbance, la phosphorescence, la chimioluminescence ou la luminescence biologique ;
(b) un détecteur d'informations optiques destiné à détecter la première et seconde information optique provenant des premières et secondes particules porteuses, dans lequel ladite première information optique est choisie parmi la fluorescence, l'absorbance, la phosphorescence, la chimioluminescence ou la luminescence biologique, et ladite seconde information optique est la lumière diffusée ;
(c) un régulateur qui différencie les premières particules porteuses, les secondes particules porteuses et les particules coexistantes sur la base de la première information optique, calcule les niveaux d'agglutination respectifs des premières et secondes particules porteuses sur la base de la seconde information optique ; et analyse si au moins une de la première et la seconde substance objet de mesure est présente dans le spécimen ou non sur la base des niveaux d'agglutination respectifs.
